# EUROPEAN PATENT SPECIFICATION

(11) **EP 2 466 675 B1**
(45) Date of publication and mention of the grant of the patent: **02.10.2013**
(21) Application number: 11193043.4
(22) Date of filing: 12.12.2011
(51) Int. Cl.: H01M 8/02, H01M 4/86, H01M 8/10, C07D 265/16

(54) **Conductive composition and method of preparation, polymer thereof, and electrode, electrolyte membrane and fuel cell including the composition or polymer**
Leitfähige Zusammensetzung und Herstellungsverfahren, Polymer davon, und Elektrode, Elektrolyt-Membran und Brennstoffzelle mit der Zusammensetzung oder Polymer
Composition conductrice et procédé de préparation, polymère associé, électrode, membrane d'électrolyte et pile à combustible incluant la composition ou le polymère

(30) Priority: 14.12.2010 KR 20100127863; 23.09.2011 US 201113242972; 27.10.2011 KR 20110110716
(43) Date of publication of application: 20.06.2012
(73) Proprietor: Samsung Electronics Co., Ltd., Suwon-si, Gyeonggi-do, 443-742 (KR)
(72) Inventor: Yoo, Dae-jong, Gyeonggi-do 449-712 (KR); Choi, Seong-woo, Gyeonggi-do 449-712 (KR); Park, So-young, Seoul (KR)
(74) Representative: Zijlstra, Robert Wiebo Johan

(56) References cited:
- EP-A1- 2 202 261
- EP-A1- 2 253 654
- US-A1- 2007 275 285

## Description

### BACKGROUND

Aspects of the present invention relate to a conductive composition, a polymer thereof, a method of preparing the composition, an electrode and an electrolyte membrane for a fuel cell including the composition or the polymer, and a fuel cell including the same.

Fuel cells are sources of future clean energy that are alternatives to fossil energy. Fuel cells also have high output density and high energy conversion efficiency, which means fuel cells are applicable in a vast range of fields such as in pollution-free vehicles, domestic power generating systems, and mobile electronic appliances such as mobile communication devices, medical equipment, and various devices for military and aerospace uses.

A fuel cell includes a cathode, an anode, and an electrolyte membrane disposed between the cathode and the anode. In the anode, to which fuel gas is supplied, oxidation of the fuel gas takes place. In the cathode, to which oxygen is supplied, reduction of oxygen occurs. Electrons generated from the reactions in the cathode and anode generate electricity, and collaterally generate heat and moisture.

Typically, such an electrode of the fuel cell includes a microporous layer consisting of carbon black and polytetrafluoroethylene. However, such an electrode may not lead to satisfactory conductivity characteristics, and thus improvement in this regard is necessary.

### SUMMARY OF THE INVENTION

Aspects of the present invention provide a conductive composition; polymers thereof; a method of preparing the conductive composition; an electrode and an electrolyte membrane for a fuel cell, each including the composition or the polymer; and a fuel cell including the same.

According to an aspect of the present invention, a composition includes at least one of a compound represented by Formula 1 below and a compound represented by Formula 2 below; and, optionally, a cross-linkable compound: 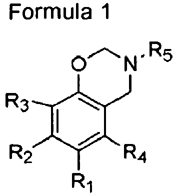 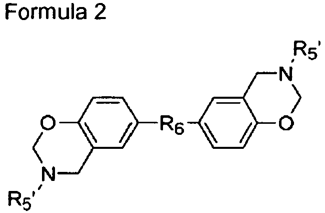
wherein, in Formulae 1 and 2, R₁ to R₄ are each independently a hydrogen atom, a substituted or unsubstituted C₁-C₂₀ alkyl group, a substituted or unsubstituted C₁-C₂₀ alkoxy group, a substituted or unsubstituted C₂-C₂₀ alkenyl group, a substituted or unsubstituted C₂-C₂₀ alkynyl group, a substituted or unsubstituted C₆-C₂₀ aryl group, a substituted or unsubstituted C₆-C₂₀ aryloxy group, a substituted or unsubstituted C₂-C₂₀ heteroaryl group, a substituted or unsubstituted C₂-C₂₀ heteroaryloxy group, a substituted or unsubstituted C₄-C₂₀ carbocyclic group, a substituted or unsubstituted C₄-C₂₀ carbocyclic oxy group, a substituted or unsubstituted C₂-C₂₀ heterocyclic group, a halogen atom, a hydroxyl group, or a cyano group;
R₆ is selected from the group consisting of a substituted or unsubstituted C₁-C₂₀ alkylene group, a substituted or unsubstituted C₂-C₂₀ alkenylene group, a substituted or unsubstituted C₂-C₂₀ alkynylene group, a substituted or unsubstituted C₆-C₂₀ arylene group, a substituted or unsubstituted C₂-C₂₀ heteroarylene group, -C(=O)-, and -SO₂-; and
R₅ and R_{5'} are each independently selected from the group consisting of a group represented by Formula 3 below, a group represented by Formula 4 below, and -S=N, 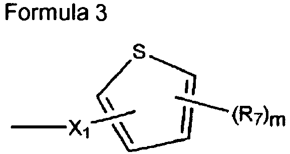 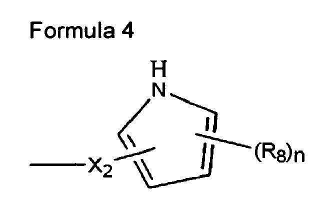
wherein, in Formulae 3 and 4, X₁ and X₂ are each independently a substituted or unsubstituted C₁-C₂₀ alkylene group, a substituted or unsubstituted C₆-C₂₀ arylene group, or a substituted or unsubstituted C₂-C₂₀ heteroarylene group;
R₇ and R₈ are each independently a hydrogen atom, a substituted or unsubstituted C₁-C₂₀ alkyl group, a substituted or unsubstituted C₆-C₂₀ aryl group, a substituted or unsubstituted C2-C20 heteroaryl group, a substituted or unsubstituted C₄-C₂₀ carbocyclic group, a substituted or unsubstituted C₂-C₂₀ heterocyclic group, a halogen atom, a hydroxyl group, an amino group, or a cyano group; and
m and n are each independently an integer from 1 to 3.

Another aspect of the present invention provides a polymer that is a polymerization product of the composition.

According to another aspect of the present invention, a method of preparing a composition includes thermally treating a mixture of a phenol compound (A) below, formaldehyde, an amine compound (B) below, an aprotic polar solvent and an organic solvent to obtain the at least one of the compounds of Formulae 1 and 2:

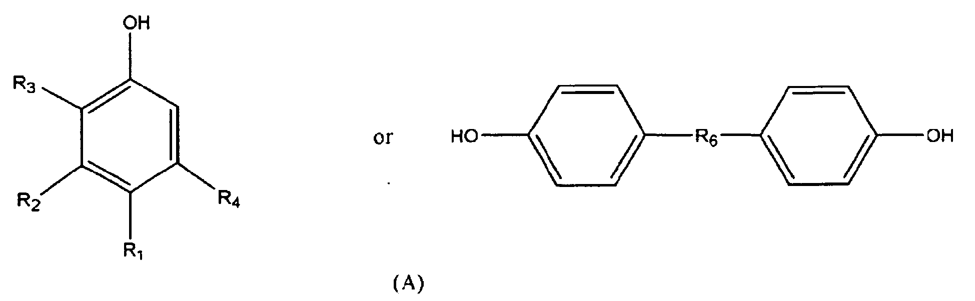

R₅NH₂ or R₅'NH₂ (B) 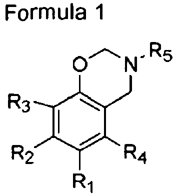 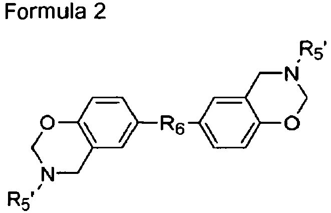
wherein, in Formulae 1 and 2 and in the formulae of the phenol compound (A) and the amine compound (B), R₁ to R₄ are each independently a hydrogen atom, a substituted or unsubstituted C₁-C₂₀ alkyl group, a substituted or unsubstituted C₁-C₂₀ alkoxy group, a substituted or unsubstituted C₂-C₂₀ alkenyl group, a substituted or unsubstituted C₂-C₂₀alkynyl group, a substituted or unsubstituted C₆-C₂₀ aryl group, a substituted or unsubstituted C₆-C₂₀ aryloxy group, a substituted or unsubstituted C₂-C₂₀ heteroaryl group, a substituted or unsubstituted C₂-C₂₀ heteroaryloxy group, a substituted or unsubstituted C₄-C₂₀ carbocyclic group, a substituted or unsubstituted C₄-C₂₀ carbocyclic oxy group, a substituted or unsubstituted C₂-C₂₀ heterocyclic group, a halogen atom, a hydroxy group, or a cyano group;
R₆ is selected from the group consisting of a substituted or unsubstituted C₁-C₂₀ alkylene group, a substituted or unsubstituted C₂-C₂₀ alkenylene group, a substituted or unsubstituted C₂-C₂₀ alkynylene group, a substituted or unsubstituted C₆-C₂₀ arylene group, a substituted or unsubstituted C₂-C₂₀ heteroarylene group, -C(=O)-, and -SO₂-; and
R₅ and R_{5'} are each independently selected from the group consisting of a group represented by Formula 3 below, a group represented by Formula 4 below, and -S≡N, 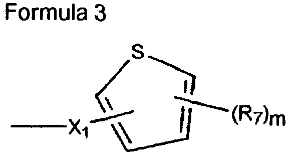 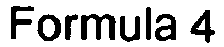 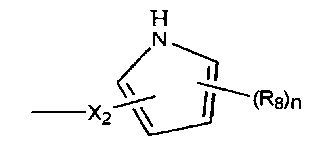
wherein, in Formulae 3 and 4, X₁ and X₂ are each independently a substituted or unsubstituted C₁-C₂₀ alkylene group, a substituted or unsubstituted C₆-C₂₀ arylene group, or a substituted or unsubstituted C₂-C₂₀ heteroarylene group;
R₇ and R₈ are each independently a hydrogen atom, a substituted or unsubstituted C₁-C₂₀ alkyl group, a substituted or unsubstituted C₆-C₂₀ aryl group, a substituted or unsubstituted C2-C20 heteroaryl group, a substituted or unsubstituted C₄-C₂₀ carbocyclic group, a substituted or unsubstituted C₂-C₂₀ heterocyclic group, a halogen atom, a hydroxyl group, an amino group, or a cyano group; and
m and n are each independently an integer from 1 to 3.

According to another aspect of the present invention, an electrode for a fuel cell includes the composition described above or the polymer described above.

According to another aspect of the present invention, an electrolyte membrane for a fuel cell includes the composition described above or the polymer described above.

According to another aspect of the present invention, a fuel cell includes: a cathode; an anode; and an electrolyte membrane disposed between the cathode and the anode, wherein at least one of the cathode, the anode and the electrolyte membrane includes the composition described above or the polymer described above.

Additional aspects and/or advantages of the invention will be set forth in part in the description which follows and, in part, will be obvious from the description, or may be learned by practice of the invention.

### BRIEF DESCRIPTION OF THE DRAWINGS

These and/or other aspects and advantages of the invention will become apparent and more readily appreciated from the following description of the embodiments, taken in conjunction with the accompanying drawings, of which:
FIG. 1 shows a nuclear magnetic resonance (NMR) spectrum of a compound represented by Formula 9 and prepared according to Synthesis Example 1;
FIG. 2 is a graph illustrating the results of measuring conductivities of microporous layers formed in Examples 1, 3, 4 and Comparative Example 1;
FIGS. 3 and 4 are scanning electron microscopic (SEM) images of the microporous layers of Example 3 and Comparative Example 1, respectively;
FIG. 5 is a graph of cell voltages of fuel cells manufactured in Example 1 and Comparative Example 1 with respect to current density; and
FIG. 6 is a graph illustrating the results of measuring conductivities of the microporous layers formed in Examples 1, 5 and Comparative Example 1.

### DETAILED DESCRIPTION

Reference will now be made in detail to the present embodiments of the present invention, examples of which are illustrated in the accompanying drawings. The embodiments are described below, to explain aspects of the present invention by referring to the figures.

An aspect of the present invention provides a composition including at least one of a compound represented by Formula 1 and a compound represented by Formula 2, and, optionally, a cross-linkable compound.

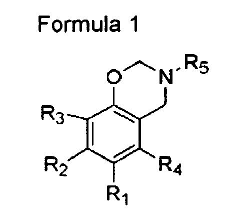

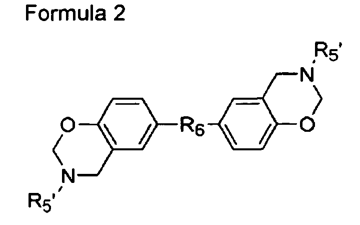

In Formulae 1 and 2, R₁ to R₄ are each independently a hydrogen atom, a substituted or unsubstituted C₁-C₂₀ alkyl group, a substituted or unsubstituted C₁-C₂₀ alkoxy group, a substituted or unsubstituted C₂-C₂₀ alkenyl group, a substituted or unsubstituted C₂-C₂₀alkynyl group, a substituted or unsubstituted C₆-C₂₀ aryl group, a substituted or unsubstituted C₆-C₂₀ aryloxy group, a substituted or unsubstituted C₂-C₂₀ heteroaryl group, a substituted or unsubstituted C₂-C₂₀ heteroaryloxy group, a substituted or unsubstituted C₄-C₂₀ carbocyclic group, a substituted or unsubstituted C₄-C₂₀carbocyclic oxy group, a substituted or unsubstituted C₂-C₂₀ heterocyclic group, a halogen atom, a hydroxyl group, or a cyano group;
R₆ is selected from the group consisting of a substituted or unsubstituted C₁-C₂₀ alkylene group, a substituted or unsubstituted C₂-C₂₀ alkenylene group, a substituted or unsubstituted C₂-C₂₀ alkynylene group, a substituted or unsubstituted C₆-C₂₀ arylene group, a substituted or unsubstituted C₂-C₂₀ heteroarylene group, -C(=O)-, and -SO₂-;
R₅ and R_{5'} are conductive functional groups, each of which is selected from the group consisting of a group represented by Formula 3, a group represented by Formula 4, and -S=N

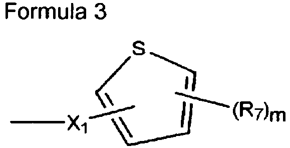

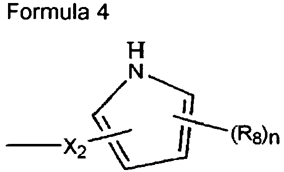

In Formulae 3 and 4, X₁ and X₂ are each independently a substituted or unsubstituted C₁-C₂₀ alkylene group, a substituted or unsubstituted C₆-C₂₀ arylene group, or a substituted or unsubstituted C₂-C₂₀ heteroarylene group;
R₇ and R₈ may be each independently a hydrogen atom, a substituted or unsubstituted C₁-C₂₀ alkyl group, a substituted or unsubstituted C₆-C₂₀ aryl group, a substituted or unsubstituted C₂-C₂₀ heteroaryl group, a substituted or unsubstituted C₄-C₂₀ carbocyclic group, a substituted or unsubstituted C₂-C₂₀ heterocyclic group, a halogen atom, a hydroxyl group, an amino group, or a cyano group;
m and n are each independently an integer from 1 to 3; and
R₅ and R₅' are each independently one of the groups represented by Formulae 5A - 5C below:

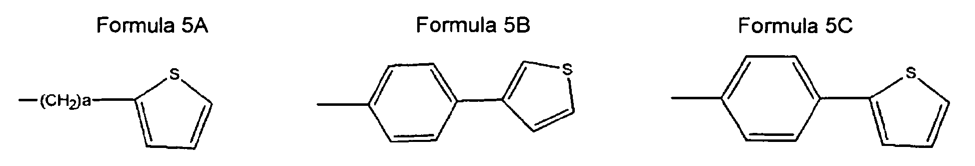

In Formula 5A, a is an integer from 1 to 5.

In Formulae 3 and 4, m and n are zero, and (R₇)₀ and (R₈)₀ are each a hydrogen atom.

Also, in Formulae 3 and 4, X₁ and X₂ may each be a methylene group, an ethylene group, a phenylene group, or the like.

When the composition includes both the compound of Formula 1 and the compound of Formula 2, the compound of Formula 2 may be from about 0.1 parts to about 100 parts by weight based on 100 parts by weight of the compound of Formula 1.

Another aspect of the present invention provides a polymer that is a polymerization product of the composition described above.

The compounds of Formulae 1 and 2 may be resistant to chemicals, heat, and acid, and may have conductive characteristics due to the conductive functional groups therein.

The composition including at least one of the compounds of Formulae 1 and 2, and the polymer that is a polymerization product of the composition may be used when forming electrodes of fuel cells. The composition may further include a cross-linkable compound.

When used in a microporous layer, the composition and the polymer therefrom may improve the dispersibility and binding strength of components in the microporous layer, thereby preventing the microporous layer from cracking at a non-uniform thickness. The microporous layer may be used to form an electrode with good conductive characteristics.

Examples of the compound of Formula 2 include compounds represented by Formulae 6 to 8 below:

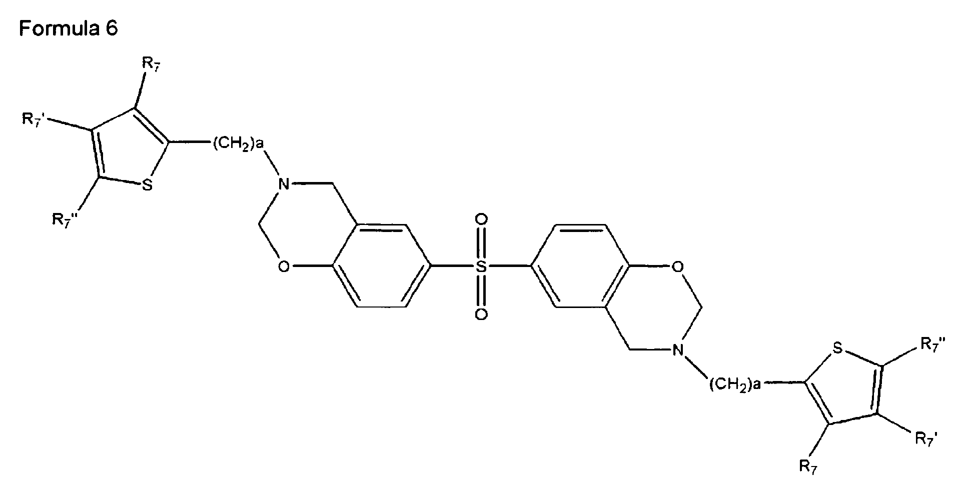

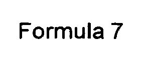

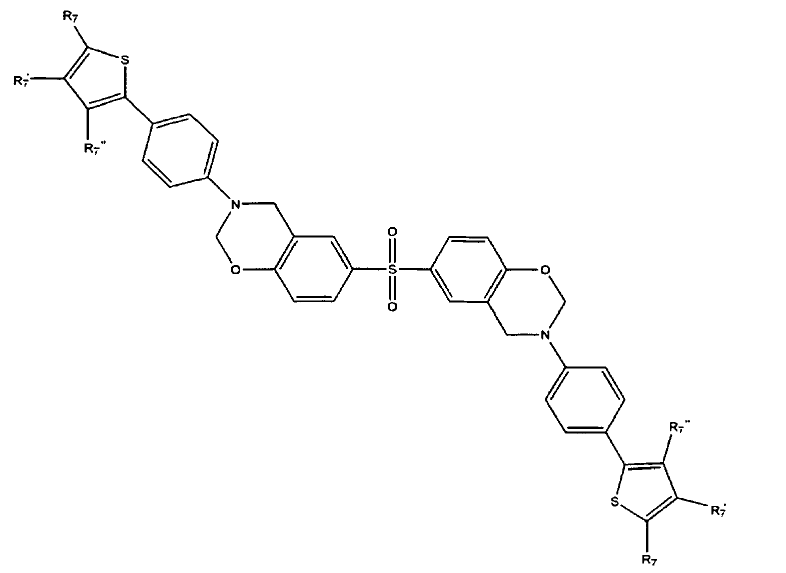

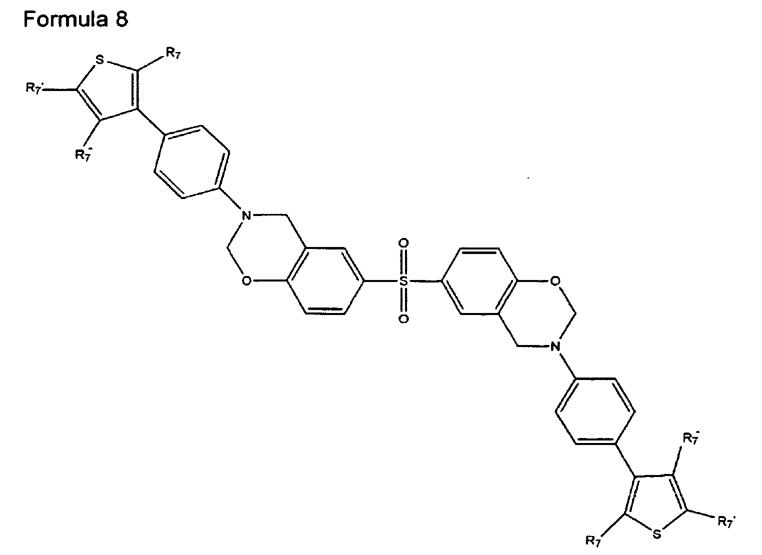

In Formulae 6-8, R₇, R₇' and R₇" are each independently a hydrogen atom, a C₁-C₂₀ alkyl group, a C₆-C₂₀ aryl group, a C₂-C₂₀ heteroaryl group, a C₂-C₂₀ heteroaryloxy group, a C₄-C₂₀ carbocyclic group, a C₂-C₂₀ heterocyclic group, a halogen atom, a hydroxyl group, or a cyano group; and in Formula 6, a is an integer from 1 to 5.

The compound of Formula 2 may include one of compounds represented by Formulae 9-11 and Formula 19 below.

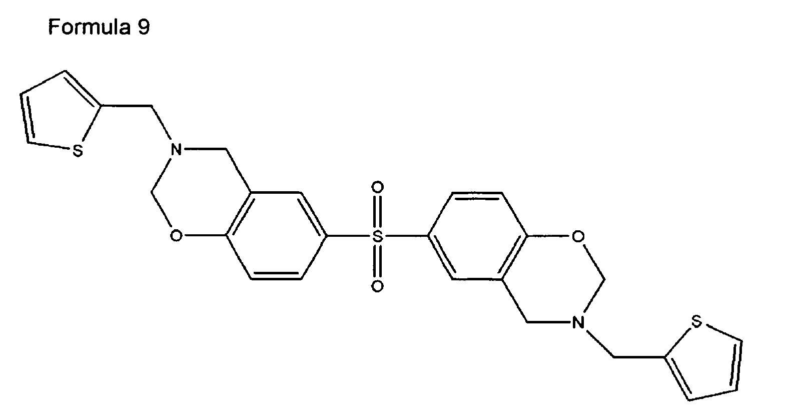

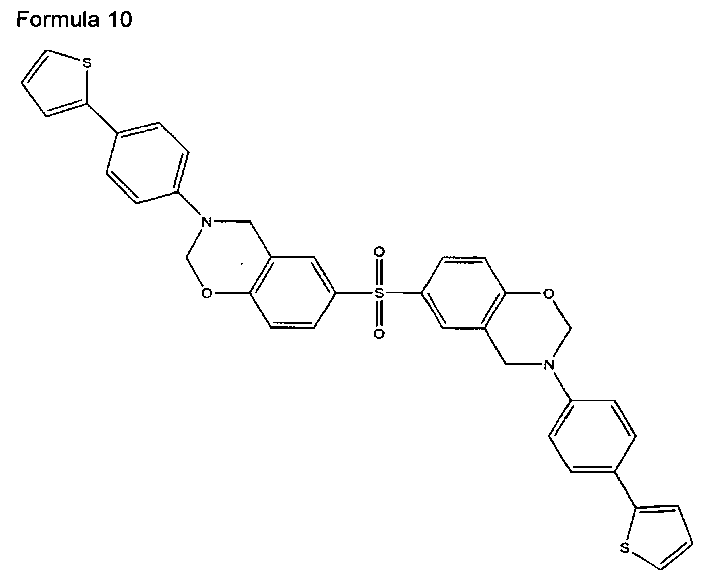

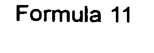

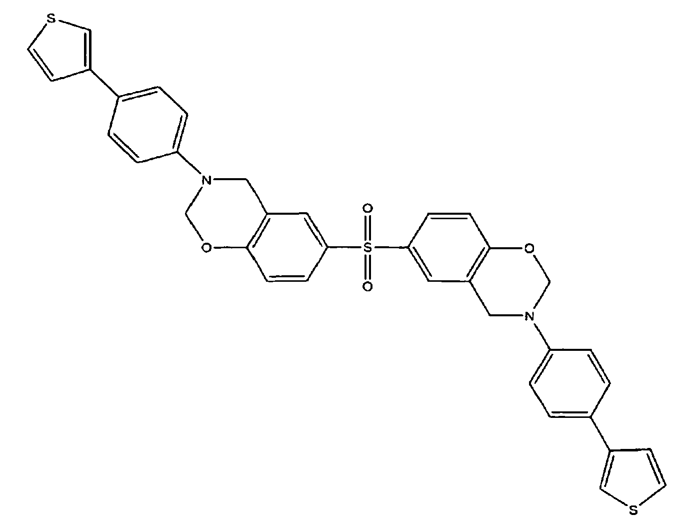

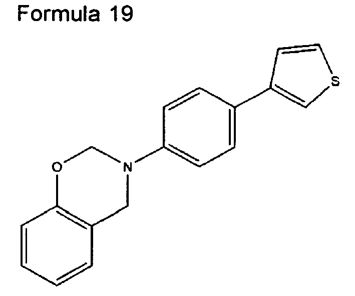

The cross-linkable compound may include any compound with a functional group which may cross-link with at least one of the compounds of Formulae 1 and 2. Examples of the cross-linkable compound include any nitrogen-containing aromatic compound, including five-membered cyclic, nitrogen-containing aromatic compounds, and six-membered cyclic, nitrogen-containing aromatic compounds, such as polypyrimidine.

The cross-linkable compound may be at least one material selected from the group consisting of a polyazole-based material, polyoxazole and polyimide.

When a polyazole-based material is used as the cross-linkable compound, a final product may be a graft copolymer obtained from graft polymerization of a polymer of at least one of the compounds of Formulae 1 and 2, and the polyazole-based material. As used herein, the term "a polymerization product of at least one of the compounds of Formulae 1 and 2, and a polyazole-based material" is in reference to the graft copolymer described above.

The polyazole-based material indicates a polymer, a repeating unit of which includes at least one aryl ring having at least one nitrogen atom. The aryl ring may include a five-membered or six-membered atom ring with one to three nitrogen atoms that may be fused to another ring, for example, another aryl ring or heteroaryl ring. In this regard, the nitrogen atoms may be substituted with oxygen, phosphorus and/or sulfur atom. Examples of the aryl ring include phenyl, naphthyl, hexahydroindyl, indanyl, tetrahydronaphthyl, and the like.

The polyazole-based material may have at least one amino group in the repeating unit as described above. In this regard, the at least one amino group may be a primary, secondary or tertiary amino group which is either part of the aryl ring or part of a substituent of the aryl ring.

The term "amino group" indicates a group with nitrogen atom covalently bonded to at least one carbon or hetero atom. The amino group may refer to, for example, -NH₂ and substituted moieties.

The term "alkyl amino group" also refers to an "alkylamino group" with nitrogen bound to at least one additional alkyl group, and "arylamino" and "diarylamino" groups with at least one or two nitrogen atoms bound to a selected aryl group.

Methods of preparing the polyazole-based material and a polymer film including the polyazole-based material are disclosed in US 2005/256296A.

Examples of the polyazole-based material include polyazole-based materials represented by Formulae 21 to 34.

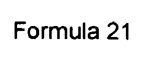

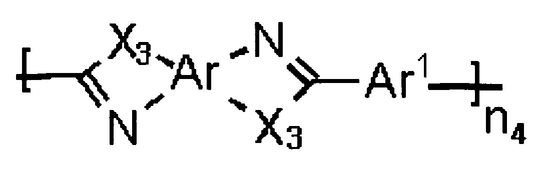

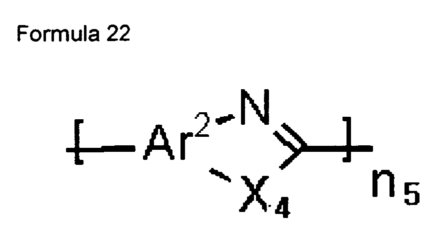

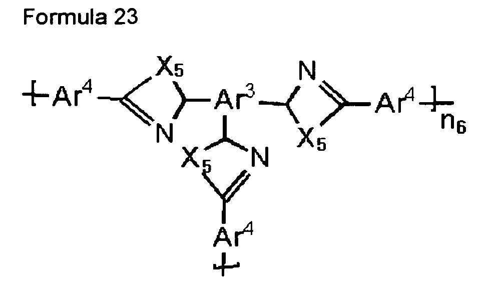

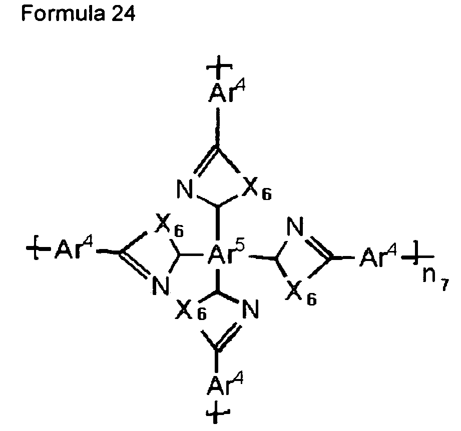

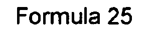

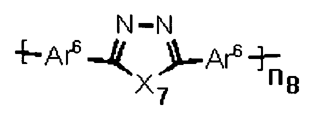

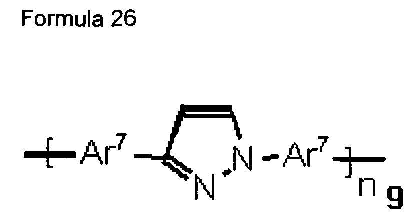

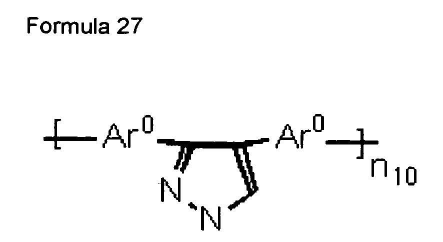

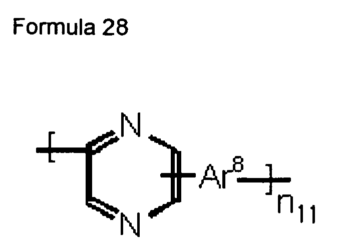

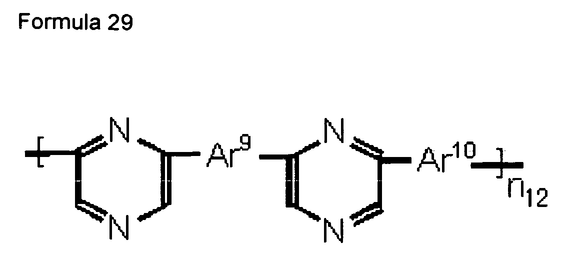

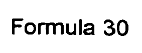

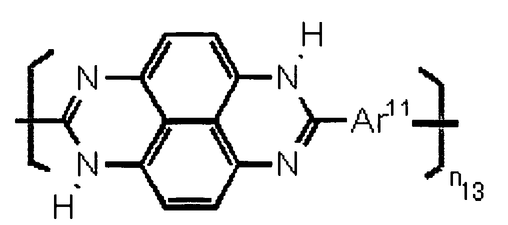

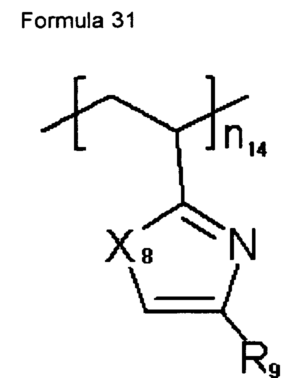

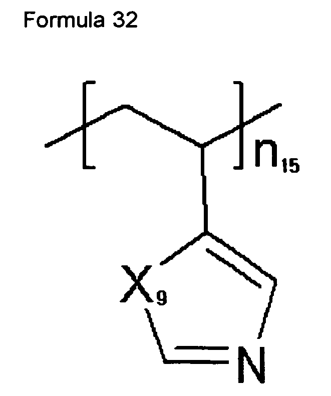

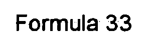

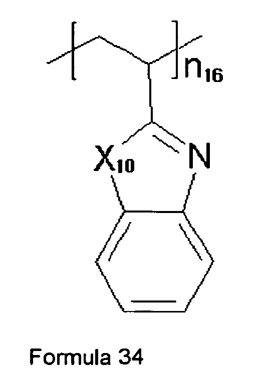

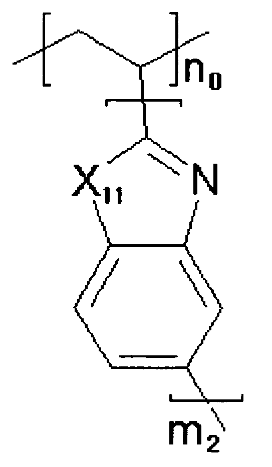

In Formulae 21 through 34, the substituents identified generically as Ar may be Ar, or Ar⁰ through Ar¹¹. Each such Ar may be identical to or different from each other such Ar, and may be a monocyclic or polycyclic C₆-C₂₀ aryl group or C₂-C₂₀ heteroaryl group. X₃ to X₁₁ may be identical to or different from each other, and may be an oxygen atom, a sulfur atom or-N(R'); and R' may be a hydrogen atom, a C₁-C₂₀ alkyl group, a C₁-C₂₀ alkoxy group or a C₆-C₂₀ aryl group;
R₉ may be identical to or different from each other, and may be a hydrogen atom, a C₁-C₂₀ alkyl group or a C₆-C₂₀ aryl group; and
n₀, n₄ to n₁₆, and m₂ may each be independently an integer of 10 or greater, and in some embodiments, may each be an integer of 100 or greater, and in some other embodiments, may each be an integer of 100 to 100,000.

Examples of the aryl or heteroaryl group include benzene, naphthalene, biphenyl, diphenylether, diphenylmethane, diphenyldimethylmethane, bisphenone, diphenylsulfone, quinoline, pyridine, bipyridine, pyridazine, pyrimidine, pyrazine, triazine, tetrazine, pyrrole, pyrazole, anthracene, benzopyrrole, benzotriazole, benzoxathiazole, benzoxadiazole, benzopyridine, benzopyrazine, benzopyridazine, benzopyrimidine, benzotriazine, indolizine, quinolizine, pyridopyridine, imidazopyrimidine, pyrazinopyrimidine, carbazole, aziridine, phenazine, benzoquinoline, phenoxazine, phenothiazine, acridizine, benzopteridine, phenanthroline and phenanthrene, wherein these aryl or heteroaryl groups may have a substituent.

Ar¹, Ar⁴, Ar⁶, Ar⁷, Ar⁸, Ar⁹, Ar¹⁰, and Ar¹¹ defined above may have any substitutable pattern. For example, if Ar¹, Ar⁴, Ar⁶, Ar⁷, Ar⁸, Ar⁹, Ar¹⁰, and Ar¹¹ are phenylene, Ar¹, Ar⁴, Ar⁶, Ar⁷, Ar⁸, Ar⁹, Ar¹⁰ and Ar¹¹ may be ortho-phenylene, meta-phenylene or para-phenylene.

The alkyl group may be a C₁-C₄ short-chain alkyl group, such as methyl, ethyl, n-propyl, i-propyl or t-butyl. The aryl group may be, for example, a phenyl group or a naphthyl group.

Examples of the substituents include a halogen atom, such as fluorine, an amino group, a hydroxyl group, and a short-chain alkyl group, such as methyl or ethyl.

Examples of the polyazole-based material include polyimidazole, polybenzothiazole, polybenzoxazole, polyoxadiazole, polyquinoxaline, polythiadiazole, polypyridine, polypyrimidine, and polytetrazapyrene.

The polyazole-based material may include a copolymer or blend including at least two units selected from the group consisting of units represented by Formulae 21 to 34 above. The polyazole-based material may include a block copolymer (di-block or tri-block), a random copolymer, a periodic copolymer or an alternating polymer including at least two units selected from the units of Formulae 21 to 34.

In some embodiments, the polyazole-based material may include only at least one of the units represented by Formulae 21 and 22.

Examples of the polyazole-based material include polymers represented by Formulae 35 to 61 below:

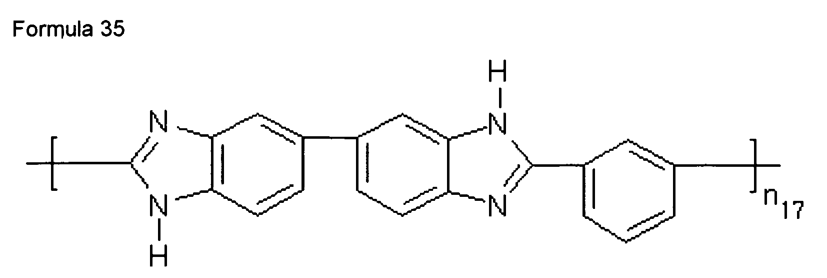

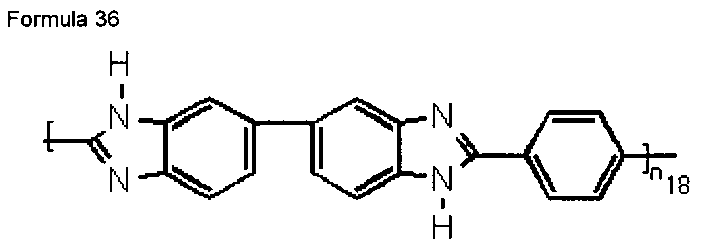

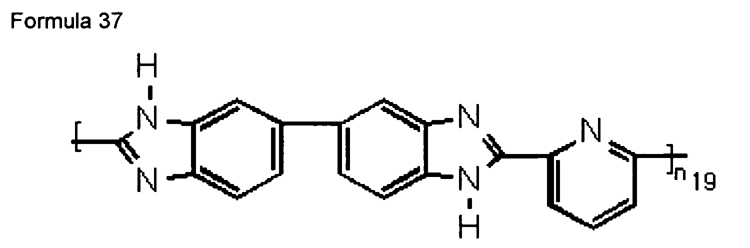

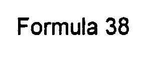

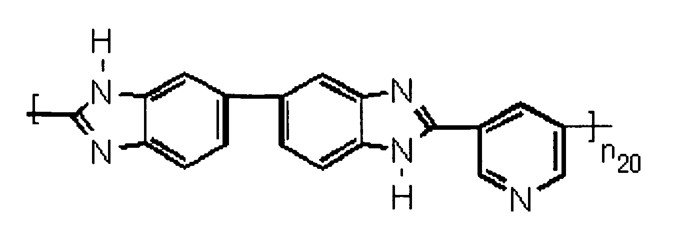

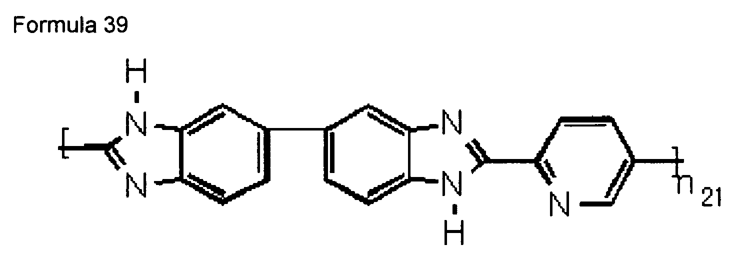

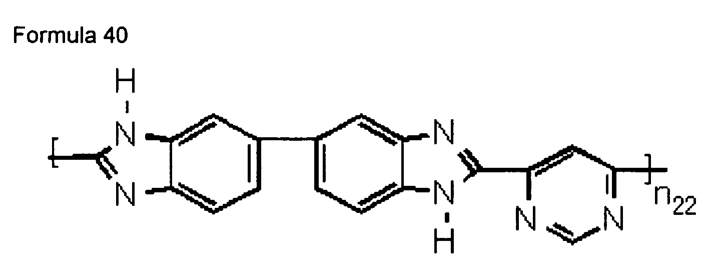

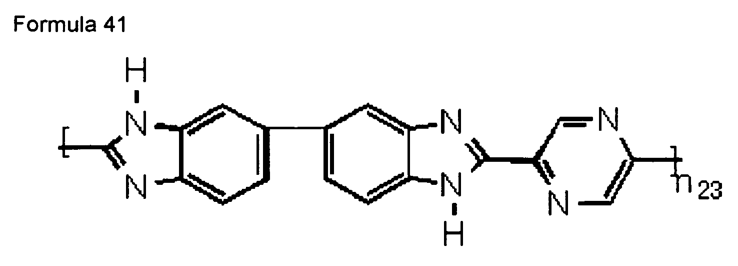

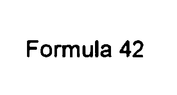

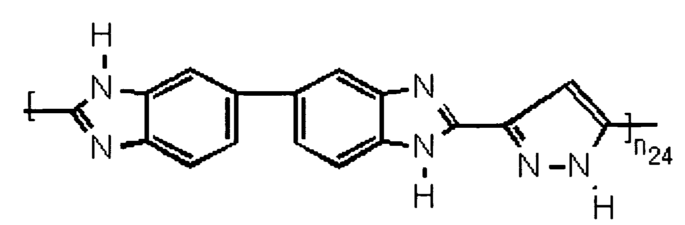

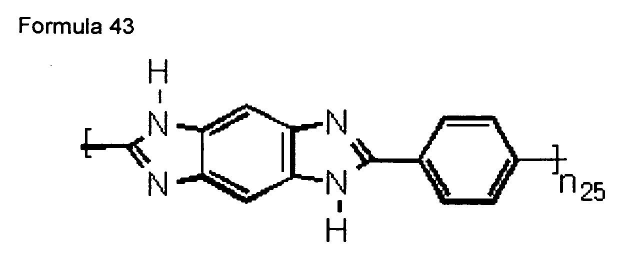

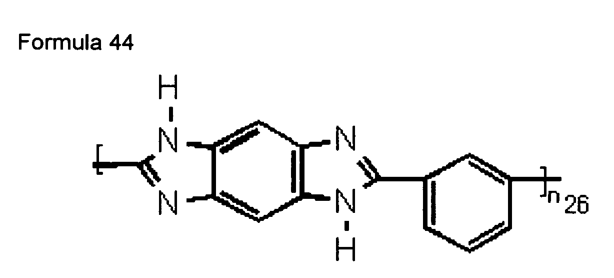

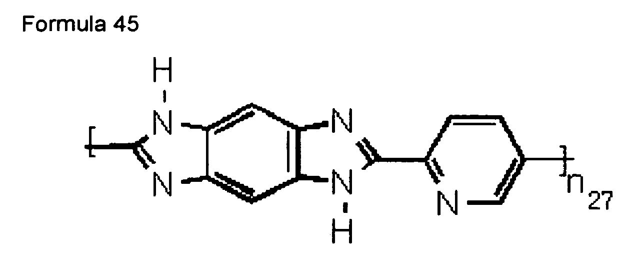

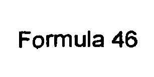

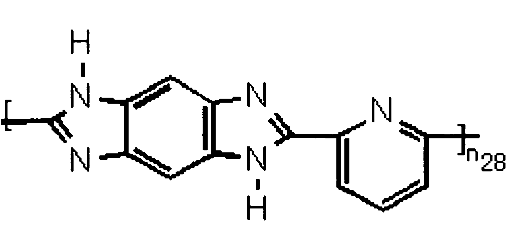

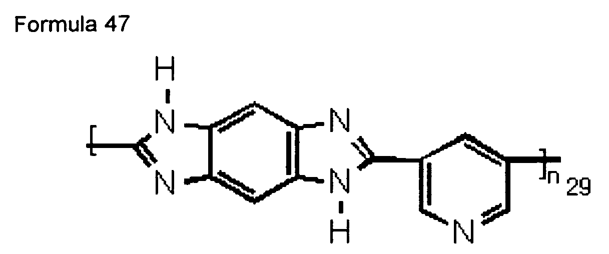

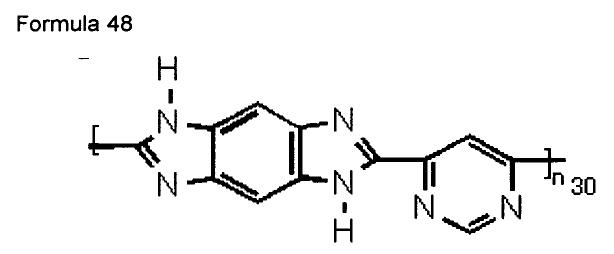

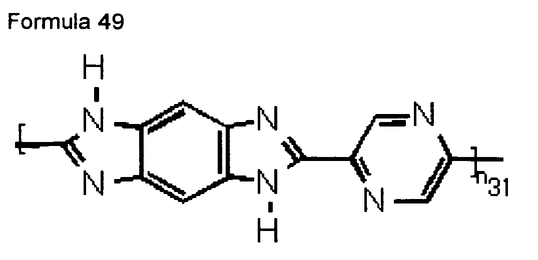

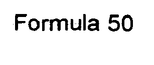

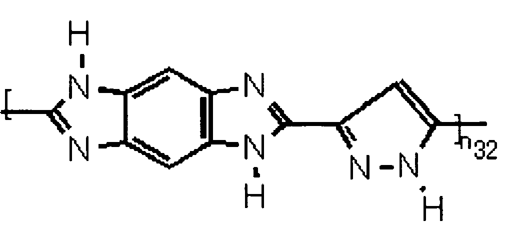

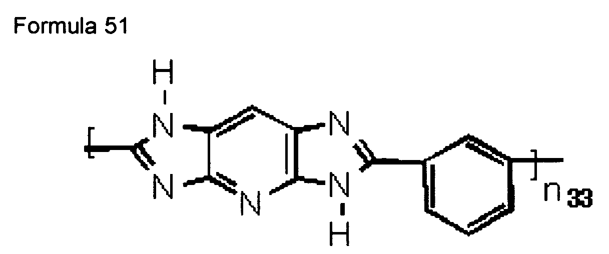

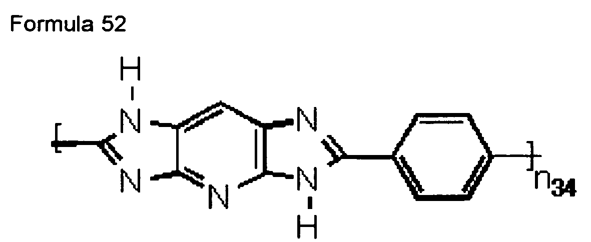

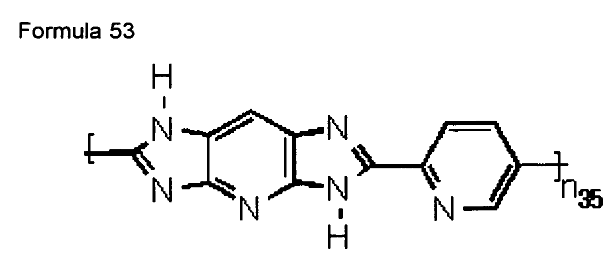

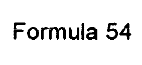

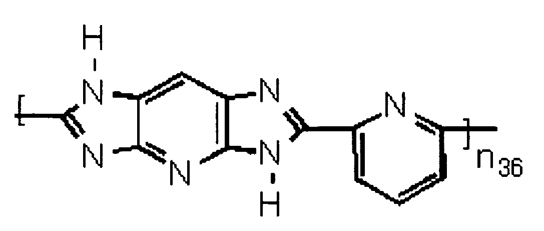

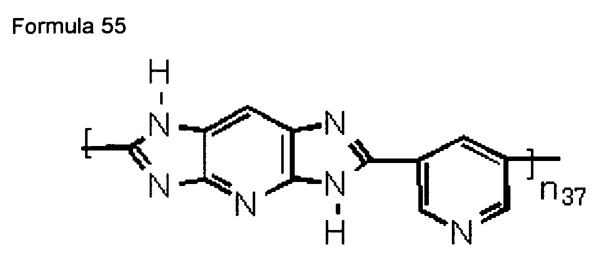

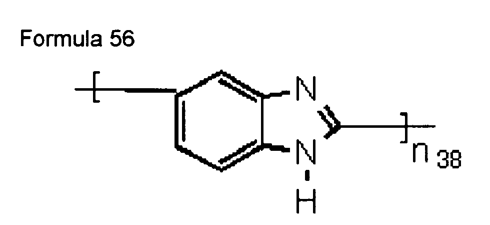

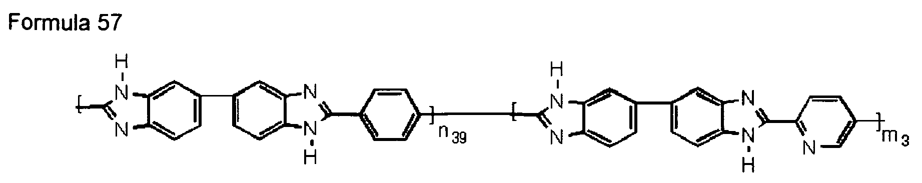

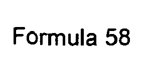

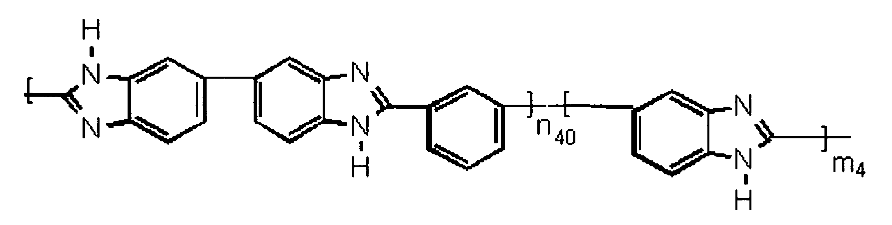

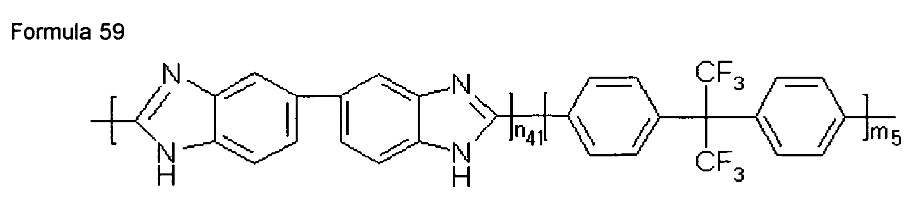

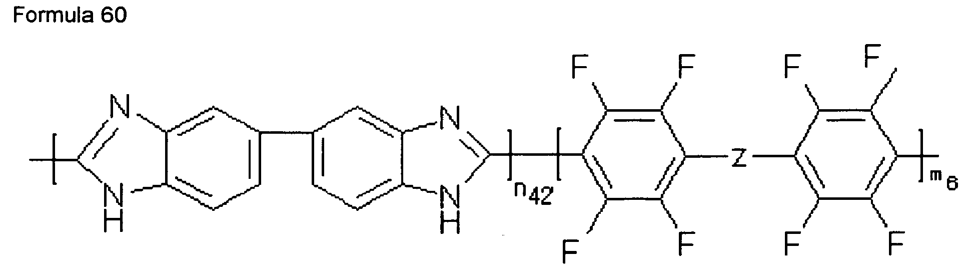

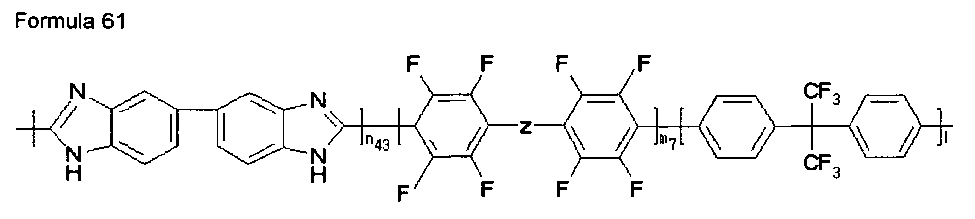

In Formulae 35 to 61, l, n₁₇ to n₄₃, and m₃ to m₇ may each be an integer of 10 or greater, and in some embodiments, may be an integer of 100 or greater, z may be a chemical bond, -(CH₂)ₛ-, -C(=O)-, -SO₂-, -C(CH₃)₂- , or -C(CF₃)₂-; and s may be an integer from 1 to 5.

The polyazole-based material may include a compound including m-polybenzimidazole (PBI) represented by Formula 12 below, or compound including p-PBI represented by Formula 13 below.

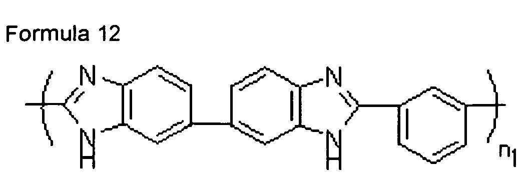

In Formula 12, n₁ is an integer of 10 or greater;

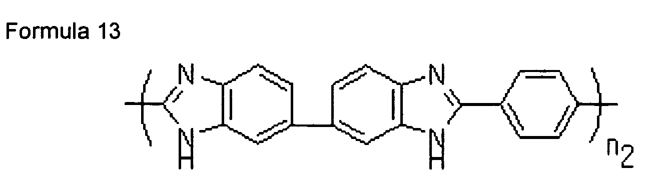

In Formula 13, n₂ is an integer of 10 or greater.

The compounds of Formulae 12 and 13 may each have a number average molecular weight of 1,000,000 or less.

For example, the polyazole-based material may include a benzimidazole-based polymer represented by Formula 14 below.

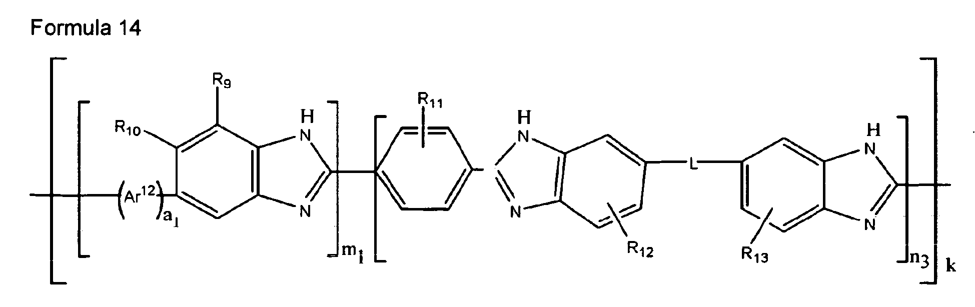

In Formula 14, R₉ and R₁₀ are each independently a hydrogen atom, an unsubstituted or substituted C₁-C₂₀ alkyl group, an unsubstituted or substituted C₁-C₂₀ alkoxy group, an unsubstituted or substituted C₆-C₂₀ aryl group, an unsubstituted or substituted C₆-C₂₀ aryloxy group, an unsubstituted or substituted C₃-C₂₀ heteroaryl group, or an unsubstituted or substituted C₃-C₂₀ heteroaryloxy group, wherein R₉ and R₁₀ may be linked to form a C₄-C₂₀ carbocyclic group or a C₃-C₂₀ heterocyclic group;
Ar¹² is a substituted or unsubstituted C₆-C₂₀ arylene group or a substituted or unsubstituted C₃-C₂₀ heteroarylene group;
R₁₁ to R₁₃ are each independently a mono- or a multi-substituted substituent selected from the group consisting of a hydrogen atom, a substituted or unsubstituted C₁-C₂₀ alkyl group, a substituted or unsubstituted C₁-C₂₀ alkoxy group, a substituted or unsubstituted C₆-C₂₀ aryl group, a substituted or unsubstituted C₆-C₂₀ aryloxy group, a substituted or unsubstituted C₃-C₂₀ heteroaryl group, and a substituted or unsubstituted C₃-C₂₀ heteroaryloxy group.
L represents a linker selected from the group consisting of a single bond, a substituted or unsubstituted C₁-C₂₀ alkylene group, a substituted or unsubstituted C₂-C₂₀ alkenylene group, a substituted or unsubstituted C₂-C₂₀ alkynylene group, a substituted or unsubstituted C₆-C₂₀ arylene group, a substituted or unsubstituted C₂-C₂₀ heteroarylene group, - C(=O)-, and -SO₂;
m₁ is from 0.01 to 1;
a₁ is 0 or 1;
n₃ is a number from 0 to 0.99; and
k is a number from 10 to 250.

The benzimidazole-based polymer may include a compound represented by Formula 15 below or a compound represented by Formula 16 below:

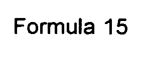

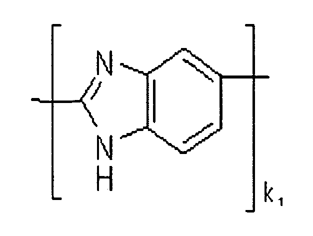

In Formula 15, k₁ represents the degree of polymerization and is a number from 10 to 300.

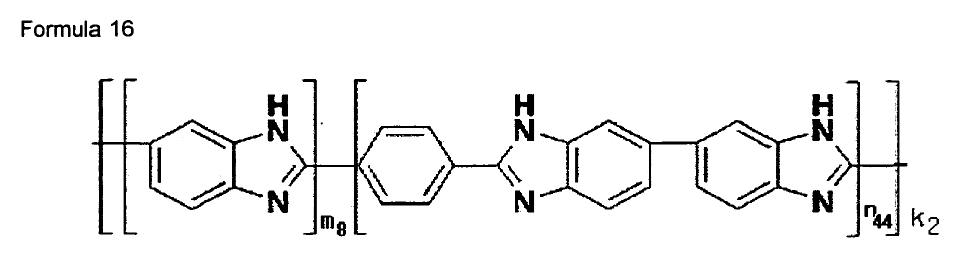

In Formula 16, m₈ is a number from 0.01 to 1, and in some embodiments, may be 1 or a number from 0.1 to 0.9; and n₄₄ is a number from 0 to 0.99, and in some embodiments, may be 0 or a number from 0.1 to 0.9; and
k₂ is a number from 10 to 250.

When at least one of the compounds of Formulae 1 and 2 is polymerized with the polyazole-based compound, the amount of the cross-linkable compound may be from about 5 parts to about 210 parts by weight, and in some embodiments, may be from about 40 parts to about 210 parts by weight based on 100 parts by weight of the at least one of the compounds of Formulae 1 and 2. When the amount of the cross-linkable compound is within these ranges, proton conductivity of a polymer obtained from the polymerization may be good.

Hereinafter, a method of preparing the composition including at least one of the compounds of Formulae 1 and 2, according to an embodiment of the present invention, will be described. First, at least one of the compounds of Formulae 1 and 2 is synthesized. Subsequently, if required, the compound of Formula 1 obtained according to the above-described process and the compound of Formula 2 may be mixed together to obtain a target composition. The amount of the compound of Formula 2 may be from about 0.01 parts to about 100 parts by weight based on 100 parts by weight of the compound of Formula 1.

A method of preparing at least one of the compounds of Formula 1 and 2 will now be described below. A phenol compound (A), formaldehyde, and an amine compound (B) are mixed together, and are then thermally treated.

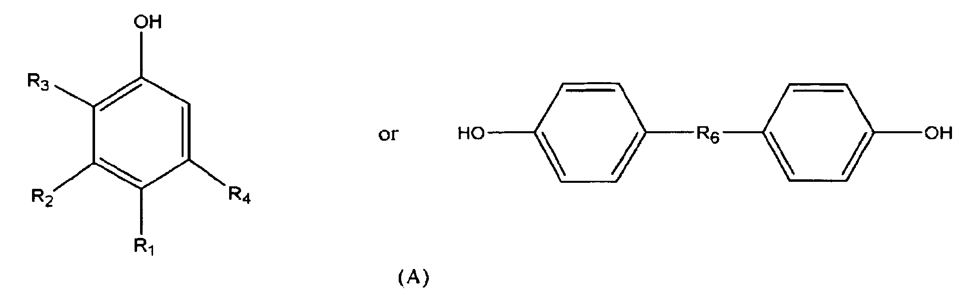

R₅NH₂ or R₅'NH₂ (B)

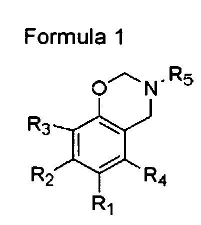

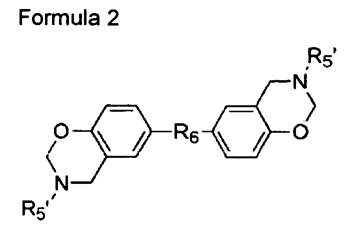

In Formulae 1 and 2 and the formulae of the phenol compound (A) and the amine compound (B), R₁ to R₄ are each independently a hydrogen atom, a substituted or unsubstituted C₁-C₂₀ alkyl group, a substituted or unsubstituted C₁-C₂₀ alkoxy group, a substituted or unsubstituted C₂-C₂₀ alkenyl group, a substituted or unsubstituted C₂-C₂₀ alkynyl group, a substituted or unsubstituted C₆-C₂₀ aryl group, a substituted or unsubstituted C₆-C₂₀ aryloxy group, a substituted or unsubstituted C₂-C₂₀ heteroaryl group, a substituted or unsubstituted C₂-C₂₀ heteroaryloxy group, a substituted or unsubstituted C₄-C₂₀ carbocyclic group, a substituted or unsubstituted C₄-C₂₀ carbocyclic oxy group,a substituted or unsubstituted C₂-C₂₀ heterocyclic group, a halogen atom, a hydroxyl group, or a cyano group;
R₆ is selected from the group consisting of a substituted or unsubstituted C₁-C₂₀ alkylene group, a substituted or unsubstituted C₂-C₂₀alkenylene group, a substituted or unsubstituted C₂-C₂₀alkynylene group, a substituted or unsubstituted C₆-C₂₀ arylene group, a substituted or unsubstituted C₂-C₂₀ heteroarylene group, -C(=O)-, and -SO₂-;
R₅ and R_{5'} are conductive functional groups, each of which is independently selected from among a group represented by Formula 3, a group represented by Formula 4, and -S≡N,

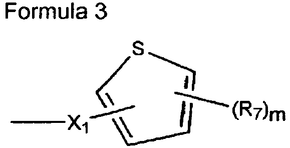

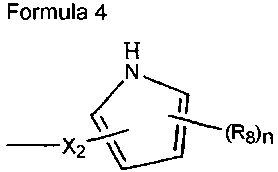

In Formulae 3 and 4, X₁ and X₂ may each be independently a substituted or unsubstituted C₁-C₂₀ alkylene group, a substituted or unsubstituted C₆-C₂₀ arylene group, or a substituted or unsubstituted C₂-C₂₀ heteroarylene group;
R₇ and R₈ may each be independently a hydrogen atom, a substituted or unsubstituted C₁-C₂₀ alkyl group, a substituted or unsubstituted C₆-C₂₀ aryl group, a substituted or unsubstituted C2-C20 heteroaryl group, a substituted or unsubstituted C₄-C₂₀ carbocyclic group, a substituted or unsubstituted C₂-C₂₀ heterocyclic group, a halogen atom, a hydroxyl group, an amino group, or a cyano group; and
m and n are each independently an integer from 1 to 3.

When the phenol compound (A), the formaldehyde, and the amine compound (B) are mixed together, an aprotic polar solvent and an organic solvent may be added. The order of adding and mixing the phenol compound (A), formaldehyde, the amine compound (B), the aprotic polar solvent, and the organic solvent is not specifically limited. The order of adding the aprotic polar solvent and the organic solvent to the phenol compound (A), formaldehyde, and the amine compound (B) is also not specifically limited. In one embodiment, the aprotic polar solvent and the organic solvent may be added to a mixture of formaldehyde and the amine compound (B), and then the phenol compound (A) may be added thereto and the combination mixed together.

The addition of the aprotic polar solvent and the organic solvent as described above enables the phenol compound (A), the formaldehyde, and the amine compound (B) to be uniformly mixed, obtaining at least one of the compounds of Formulae 1 and 2 with a high yield. If the aprotic polar solvent is not used to mix the phenol compound (A), formaldehyde, and the amine compound (B) together, it may be difficult to obtain at least one of the compounds of Formulae 1 and 2.

The aprotic polar solvent may include at least one solvent selected from the group consisting of N, N'-dimethylformamide, N,N'-dimethylacetamide, N-methylpyrrolidone, tetramethylenesulfone, 1,2-dimethyl-2-imidazolidinone, and N-methylformamide.

The organic solvent may include 1,4-dioxane, chloroform, dichloromethane, tetrahydrofuran (THF), a benzene-based solvent, or a mixture thereof.

In one embodiment, the organic solvent may include a benzene-based solvent. The benzene-based solvent may include at least one solvent selected from among benzene, toluene and xylene.

The mixing ratio of the aprotic polar solvent to the organic solvent may be from about 1:9 to about 9:1 by volume, and in some embodiments, may be from about 1:5 to about 5:1 by volume. In one embodiment, the mixing ratio of the aprotic polar solvent to the organic solvent may be about 1:3 by volume.

When the phenol compound (A), formaldehyde, and the amine compound (B) are mixed together, at least one catalyst selected from among p-toluenesulfonic acid, phosphorus pentachloride (PCl₅), and phosphoryl chloride (POCl₃) may be further added. The amount of the catalyst may be from about 10⁻⁶ mol to about 5×10⁻¹ mol based on 1 mol of the phenol compound (A). When the amount of the catalyst is within this range, at least one of the compounds of Formulae 1 and 2 may be obtained with a high yield.

The thermal treatment may be performed at a temperature of about 80 °C to about 250 °C.

The amount of the formaldehyde may be from about 2 moles to about 5 moles, and in some embodiments, may be about 4.4 moles, based on 1 mol of the phenol compound (A). The amount of the amine compound (B) may be from about 1 mol to about 4 moles, and in some embodiments, may be about 2.2 moles, based on 1 mol of the phenol compound (A). When the amounts of the formaldehyde and the amine compound (B) are within these respective ranges, at least one of the compounds of Formulae 1 and 2 may be obtained with a high yield.

Hereinafter, a method of preparing a polymer of at least one of the compounds of Formulae 1 and 2, according to an embodiment of the present invention, will be described. In this regard, a non-limiting exemplary method of preparing a compound represented by Formula 6 below, which is an example of the compound of Formula 2, will be described.

The compound represented by Formula 6 below may be prepared through reaction of the phenol compound (A), the formaldehyde, and the amine compound (B), as illustrated in Reaction Scheme 1 below. The reaction is performed under conditions in which a mixed solvent of an aprotic polar solvent and an organic solvent is used.

When the phenol compound (A), the formaldehyde, and the amine compound (B) react, one catalyst selected from among p-toluenesulfonic acid, phosphorus pentachloride (PCl₅), and phosphoryl chloride (POCl₃) may be further added.

The same kinds and same amounts of aprotic polar solvents and organic solvents as described above may be used. The same amount of catalyst as described above may be used.

The reaction temperature may be from about 80 °C to about 150 °C.

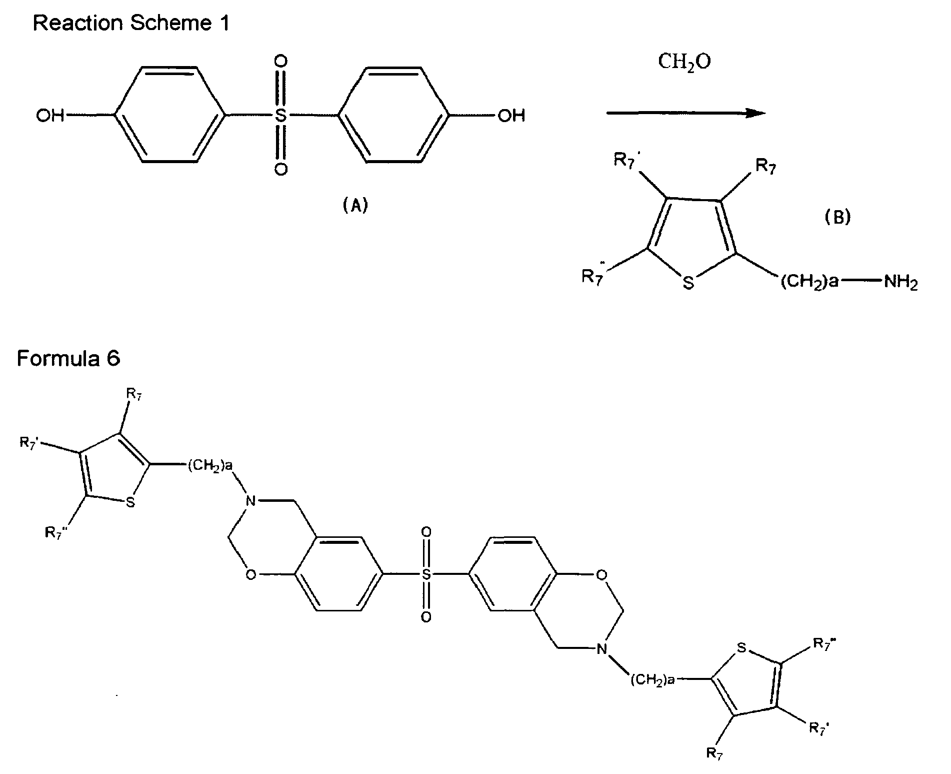

In Reaction Scheme 1 and Formula 6 above, R₇, R₇', and R₇" are each independently a hydrogen atom, a C₁-C₂₀ alkyl group, a C₆-C₂₀ aryl group, a C₂-C₂₀ heteroaryl group, a substituted or non-substituted C₂-C₂₀ heteroaryloxy group, a C₄-C₂₀ carbocyclic group, a C₂-C₂₀ heterocyclic group, a halogen atom, a hydroxyl group, an amino group, or a cyano group; and a is an integer from 1 to 5.

In Reaction Scheme 1 above, the amount of the formaldehyde may be from about 2 moles to about 5 moles, and in some embodiments, may be about 4.4 moles, based on 1 mol of the phenol compound (A). The amount of the amine compound (B) may be from about 1 mol to about 4 moles, and in some embodiments, may be about 2.2 moles, based on 1 mol of the phenol compound (A). When the amounts of the formaldehyde and the amine compound (B) are within these respective ranges, the compound of Formula 6 may be obtained with a high yield.

The compound of Formula 1 above may also be synthesized in a similar method in which the compound of Formula 6, an example of the compound of Formula 2, is synthesized.

In some embodiments the polymer that is a polymerization product of the composition may include a compound represented by Formula 17 below. The compound of Formula 17 may be part of the polymer.

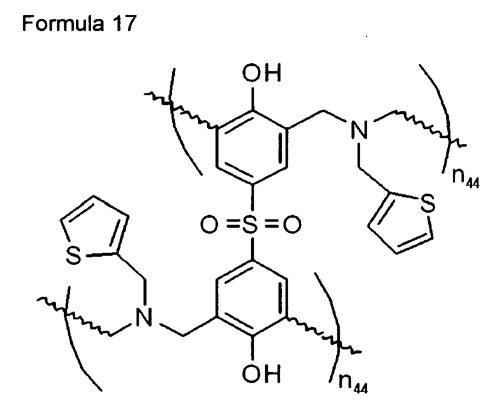

In Formula 17, n₄₄ is an integer from 5 to 200.

At least one of the composition and the polymer obtained therefrom are useful as an electrode additive for fuel cells. An electrode for fuel cells may have a structure including a catalyst layer and a gas diffusion layer (GDL). A microporous layer may be further disposed between the GDL and the catalyst layer. The microporous layer may reduce contact resistance of the gas diffusion layer with respect to the catalyst layer and may facilitate gas permeation and discharge of water, which is a byproduct from the reaction.

According to another aspect of the present invention, an electrode for fuel cells includes a gas diffusion layer and a microporous layer, and a catalyst layer; and the at least one of the gas diffusion layer, the microporous layer and the catalyst layer comprises at least one of the compositions described above and the polymer that is a polymerization product of the compositions. For example, the at least one of the gas diffusion layer, the microporous layer and the catalyst layer contains the polymer.

According to another aspect of the present invention, an electrode for fuel cells includes at least one of a microporous layer and a catalyst layer, for example, a microporous layer or a catalyst layer that contains at least one of the compositions described above and the polymer that is a polymerization product of at least one of the compositions represented by Formulae 1 and 2. For example, the microporous layer or the catalyst layer contains the polymer.

According to another aspect of the present invention, an electrode for fuel cells includes a gas diffusion layer and a microporous layer, and a catalyst layer; and the gas diffusion layer and the microporous layer contain at least one of the compositions described above and the polymer that is a polymerization product of at least one of the compositions represented by Formulae 1 and 2. For example, the gas diffusion layer and the microporous layer contain the polymer.

The composition including at least one of the compounds of Formulae 1 and 2 may further include a cross-linkable compound.

First, a method of manufacturing an electrode for fuel cells that includes a catalyst layer, a microporous layer, and a GDL will be described in detail. For an electrode that does not include a microporous layer, the catalyst layer may be directly formed on the GDL.

The microporous layer may include a conductive material, and at least one of the compositions described above and the polymer that is a polymerization product of the composition.

At least one of the composition and the polymer obtained therefrom may be from about 0.1 parts to about 0.5 parts by weight based on 1 part by weight of the conductive material. When the amount of the at least one of the composition and the polymer is within this range, the conductive material may be effectively bound in the conductive layer, thus reducing resistance of the electrode. The conductive material may include carbon black, graphite, glass carbon, activated charcoal, carbon fiber, activated carbon, carbon aerogel, or a mixture thereof.

A method of forming the microporous layer is as follows. First, the conductive material is dispersed or dissolved in a solvent to prepare a mixture A. Examples of the solvent include ethylene glycol, methanol, ethanol, isopropanol, N-methylpyrolidone (NMP), N, N-dimethylacetamide (DMAc), and the like. The amount of the solvent may be from about 200 parts to about 500 parts by weight based on 100 parts by weight of the conductive material. When the amount of the solvent is within this range, good workability in forming the microporous layer may be achieved.

Meanwhile, at least one of the compounds of Formulae 1 and 2 is dispersed or dissolved in a solvent to obtain a mixture B. A cross-linkable compound may be further added to the mixture B. The solvent may include N-methylpyrolidone (NMP), N, N-dimethylacetamide (DMAc), or the like.

The amount of the solvent may be from about 1,000 parts to about 2,000 parts by weight based on 100 parts by weight of at least one of the compounds of Formulae 1 and 2. When the amount of the solvent is within this range, this may lead to good workability in forming the microporous layer.

The mixture A and the mixture B are mixed together to obtain a mixture, which is coated on the GDL. Non-limiting examples of the coating method include bar coating, tape casting, screen printing, and the like.

A porous carbon support may be used as the GDL. Examples of the porous carbon support include carbon paper, carbon cloth, and the like.

The resulting structure with the mixture coated on the GDL is optionally thermally heated to form the microporous layer. The thermal treatment temperature may be from about 200 °C to about 270 °C.

The microporous layer formed according to the processes described above may have a thickness of about 30 nm to about 80 nm, and an electrical resistance of about 12 Ωcm²/cm to about 14 Ωcm²/cm.

Then, the catalyst layer is formed on the microporous layer, thereby completing the manufacture of the electrode for fuel cells. The catalyst layer includes a catalyst. The catalyst may be platinum (Pt), an alloy or a mixture of Pt and at least one metal selected from the group consisting of gold (Au), palladium (Pd), rhodium (Ru), iridium (Ir), ruthenium (Ru), tin (Sn), molybdenum (Mo), cobalt (Co), and chromium (Cr). The Pt, the alloy, or the mixture may be supported on a carbonaceous support.

The catalyst layer of the electrode may further include a binder. The binder may include at least one selected from the group consisting of poly(vinylidenefluoride), polytetrafluoroethylene (PTFE), a tetrafluoroethylene-hexafluoropropylene copolymer, and polyurethane.

In some embodiments the binder may include at least one of the composition described above and the polymer obtained therefrom, wherein the composition includes at least one of the compounds of Formulae 1 and 2, as described above.

The composition may further include a binder. As described above, when at least one of the compositions described above, including at least one of the compounds of Formulae 1 and 2, and the polymer that is a polymerization product of the compositions is used to form the microporous layer and the catalyst layer, a uniform electrode with increased dispersibility and binding strength of the conductive material may be formed.

The amount of the binder may be from about 0.001 parts to about 0.5 parts by weight, and in some embodiments, may be from about 0.01 parts to about 0.1 parts by weight, based on 1 part by weight of the catalyst. When the amount of the binder is within these ranges, the electrical conductivity of the electrode may be improved with increased binding strength of the conductive material in the electrode.

The catalyst layer of the electrode for fuel cells may be manufactured using any of various known methods. A non-limiting exemplary method of forming the catalyst layer is as follows. First, a catalyst is dispersed in a solvent. The solvent may include N-methylpyrrolidone (NMP), dimethylacetamide (DMAc), or the like. The amount of the solvent may be from about 1 part to about 10 parts by weight based on 1 part by weight of the catalyst.

A mixture of a solvent and at least one of the compounds of Formulae 1 and 2 is added to the dispersion and then stirred to obtain a coating solution. A binder may be further added to the coating solution. In one embodiment, a polyazole-based material may be further added to the coating solution.

The solvent may include N-methylpyrrolidone (NMP), N,N-dimethylacetamide (DMAC), or the like. The amount of at least one of the compounds of Formulae 1 and 2 may be from about 0.001 parts to about 0.5 parts by weight based on 1 part by weight of the catalyst. When a common binder is used along with at least one of the compounds of Formulae 1 and 2, the amount of the at least one of the compounds of Formulae 1 and 2 may be from about 0.001 parts to about 0.1 parts by weight based on 1 part by weight of the catalyst. When the amount of the at least one of the compounds of Formulae 1 and 2 is within this range, the electrode may have improved conductive characteristics.

The coating solution is coated on a surface of the microporous layer, and dried, thereby completing the manufacture of the electrode. For an electrode that does not include the microporous layer, the catalyst layer may be formed directly on the GDL.

The method of coating the coating solution is not particularly limited. Examples of the coating method include coating using a doctor blade, bar coating, screen printing, and the like.

After the coating solution has been coated on the surface of the microporous layer, the resulting structure is dried at a temperature of from about 20 °C to about 150 °C to remove the solvent. The drying time may vary depending on the drying temperature. In some embodiments the drying time may be from about 10 minutes to about 120 minutes.

The catalyst layer for the electrode may be formed in another way. That is, rather than being directly coated on the surface of the microporous layer or the GDL, the coating solution may be coated on a separate support, dried, and separated from the support to prepare a catalyst layer, which may be then placed on the microporous layer or the GDL, thereby completing the manufacture of the electrode. The catalyst layer for the electrode may also be formed using at least one of the compositions described above and the polymer obtained therefrom.

A method of manufacturing a fuel cell including the electrode described above will be described below. Any electrolyte membrane that is commonly used in fuel cells may be used. Suitable examples of electrolyte membranes include a polybenzimidazole electrolyte membrane, a polybenzoxazine-polybenzimidazole copolymer electrolyte membrane, a porous PTFE membrane, a fluorosulfonic acid-base membrane, a sulfone-based hydrocarbon membrane, and an electrolyte membrane as disclosed in US Patent Application Publication No. 20070275285A.

In a similar way to the electrode, the electrolyte membrane may include the polymer obtained from the polymerization of the composition including at least one of the compounds of Formulae 1 and 2.

The electrolyte membrane may further include a proton conductor. Examples of the proton conductor include polyphosphoric acid, phosphorous acid (H₃PO₃), orthophosphoric acid (H₃PO₄), pyrophosphoric acid (H₄P₂O₇), triphosphoric acid (H₅P₃O₁₀), metaphosphoric acid, and a derivative thereof. The concentration of the proton conductor may be from about 80 wt% to about 98 wt%, and in some embodiments, may be 80 wt%, 90 wt%, 95 wt%, or 98 wt%.

An electrolyte membrane may be manufactured using the polymer obtained from polymerization of the at least one of the compounds of Formulae 1 and 2, according to a method disclosed in US Patent Application Publication No. 20070275285A.

Substituents in the formulae above may be defined as follows.

As used herein, the term "alkyl" refers to a fully saturated branched or unbranched (or straight chain or linear) hydrocarbon moiety. Examples of the alkyl group used herein include, but are not limited to, methyl, ethyl, n-propyl, iso-propyl, n-butyl, sec-butyl, iso-butyl, tert-butyl, n-pentyl, isopentyl, neopentyl, n-hexyl, 3-methylhexyl, 2,2-dimethylpentyl, 2,3-dimethylpentyl, and n-heptyl.

At least one hydrogen atom of the alkyl group may be substituted with a halogen atom, a C₁-C₂₀ alkyl group substituted with a halogen atom (for example, CCF₃, CHCF₂, CH₂F and CCl₃), a C₁-C₂₀ alkoxy, a C₂-C₂₀ alkoxyalkyl, a hydroxy group , a nitro group , a cyano group , an amino group , an amidino group , a hydrazine group , a hydrazone group, a carboxyl group or a salt thereof , a sulfonyl group , a sulfamoyl , a sulfonic acid group or a salt thereof , a phosphoric acid or a salt thereof , a C₁-C₂₀ alkyl group , a C₂-C₂₀ alkenyl group , a C₂-C₂₀ alkynyl group , a C₁-C₂₀ heteroalkyl group , a C₆-C₂₀ aryl group , a C₆-C₂₀ arylalkyl group , a C₆-C₂₀ heteroaryl group , a C₇-C₂₀ heteroarylalkyl group , a C₆-C₂₀ heteroaryloxy group , a C₆-C₂₀ heteroaryloxyalkyl group , or a C₆-C₂₀ heteroarylalkyl group.

As used herein, the term "halogen atom" refers to fluoro, bromo, chloro, or iodo.

As used herein, the term "a C₁-C₂₀ alkyl group substituted with a halogen atom" refers to a C₁-C₂₀ alkyl group that is substituted with one or more halo groups, and unlimited examples of a C₁-C₂₀ alkyl group that is substituted with one or more halo groups are monohaloalkyl, dihaloalkyl, and polyhaloalkyl including perhaloalkyl. A monohaloalkyl has one iodo, bromo, chloro or fluoro within the alkyl group, and dihaloalky and polyhaloalkyl groups have two or more of the same halo atoms or a combination of different halo groups within the alkyl.

As used herein, the term "alkoxy" refers to alkyl-O—, wherein alkyl is defined herein above. Examples of alkoxy include, but are not limited to, methoxy, ethoxy, propoxy, 2-propoxy, butoxy, tert-butoxy, pentyloxy, hexyloxy and the like. At least one hydrogen atom of the alkoxy group may be substituted with one of the same substituents as described above in connection with the alkyl group.

The term alkoxyalkyl refers to an alkyl group, as defined above, in which the alkyl group is substituted with alkoxy. At least one hydrogen atom of the alkoxyalkyl group may be substituted with the same substituent as described above in connection with the alkyl group. The term alkoxyalkyl includes a substituted alkoxyalkyl moiety.

The term "alkenyl" refers to a branched or unbranched hydrocarbon having at least one carbon-carbon double bond. Examples of alkenyl are, but are not limited to, vinyl, allyl, butenyl, isopropenyl or isobutenyl. At least one hydrogen atom of the alkenyl group may be substituted with one of the same substituents as described above in connection with the alkyl group.

The term "alkynyl" refers to a branched or unbranched hydrocarbon having at least one carbon-carbon triple bond. Examples of alkynyl are, but are not limited to, ethynyl, butynyl, isobutynyl or isopropynyl. At least one hydrogen atom of alkynyl may be substituted with one of the same substituents as described above in connection with the alkyl group.

The term "aryl" is used alone or in combination, and refers to an aromatic hydrocarbon group having one or more rings. The term "aryl" also refers to a group in which an aromatic ring is fused to one or more cycloalkyl rings. Examples of aryl are, but are not limited to, phenyl, naphthyl, or tetrahydronaphthyl. At least one hydrogen atom of aryl may be substituted with one of the same substituents as described above in connection with the alkyl group.

The term "arylalkyl" is an alkyl substituted with aryl. Examples of arylalkyl are benzyl or phenyl-CH₂CH₂-.

The term "aryloxy" includes an -O-aryl, wherein aryl is defined herein. Examples of aryloxy are phenoxy and the like. At least one hydrogen atom of aryloxy may be substituted with the same substituent as described above in connection with the alkyl group.

The term "heteroaryl" refers to a monocyclic or bicyclic aromatic organic group that contains one or more hetero atoms selected from N, O, P, and S, and the remaining ring atoms are carbon atoms. The heteroaryl may include, for example, 1 to 5 hetero atoms, and 5 to 10 ring members. S or N may be oxidized to various oxidation states.

Typical monocyclic heteroaryl groups include thienyl, furyl, pyrrolyl, imidazolyl, pyrazolyl, thiazolyl, isothiazolyl, 1,2,3-oxadiazolyl, 1,2,4-oxadiazolyl, 1,2,5-oxadiazolyl, 1,3,4-oxadiazolyl, 1,2,3-thiadiazolyl, 1,2,4-thiadiazolyl, 1,2,5-thiadiazolyl, 1,3,4-thiadiazolyl, isothiazol-3-yl, isothiazol-4-yl, isothiazol-5-yl, oxazol-2-yl, oxazol-4-yl, oxazol-5-yl, isooxazol-3-yl, isooxazol-4-yl, isooxazol-5-yl, 1,2,4-triazol-3-yl, 1,2,4-triazol-5-yl, 1,2,3-triazol-4-yl, 1,2,3-triazol-5-yl, tetrazolyl, pyrid-2-yl, pyrid-3-yl, 2-pyrazin-2-yl, pyrazin-4-yl, pyrazin-5-yl, 2- pyrimidin-2-yl, 4- pyrimidin-2-yl, and 5-pyrimidin-2-yl.

The term "heteroaryl" also refers to a group in which a heteroaromatic ring is fused to one or more aryl, cycloaliphatic, or heterocyclic rings. Examples of bicyclic heteroaryl are indolyl, isoindolyl, indazolyl, indolizinyl, purinyl, quinolizinyl, quinolinyl, isoquinolinyl, cinnolinyl, phthalazinyl, naphthyridinyl, quinazolinyl, quinoxalinyl, phenanthridinyl, phenanthrolinyl, phenazinyl, phenothiazinyl, phenoxazinyl, benzisoquinolinyl, thieno[2,3-b]furanyl, furo[3,2-b]-pyranyl, 5H-pyrido[2,3-d]-o-oxazinyl, 1H-pyrazolo[4,3-d]-oxazolyl, 4H-imidazo[4,5-d]thiazolyl, pyrazino[2,3-d]pyridazinyl, imidazo[2,1-b]thiazolyl, imidazo[1,2-b][1,2,4]triazinyl, 7-benzo[b]thienyl, benzoxazolyl, benzimidazolyl, benzothiazolyl, benzoxapinyl, benzoxazinyl, 1H-pyrrolo[1,2-b][2]benzazapinyl, benzofuryl, benzothiophenyl, benzotriazolyl, pyrrolo[2,3-b]pyridinyl, pyrrolo[3,2-c]pyridinyl, pyrrolo[3,2-b]pyridinyl, imidazo[4,5-b]pyridinyl, imidazo[4,5-c]pyridinyl, pyrazolo[4,3-d]pyridinyl, pyrazolo[4,3-c]pyridinyl, pyrazolo[3,4-c]pyridinyl, pyrazolo[3,4-d]pyridinyl, pyrazolo[3,4-b]pyridinyl, imidazo[1,2-a]pyridinyl, pyrazolo[1,5-a]pyridinyl, pyrrolo[1,2-b]pyridazinyl, imidazo[1,2-c]pyrimidinyl, pyrido[3,2-d]pyrimidinyl, pyrido[4,3-d]pyrimidinyl, pyrido[3,4-d]pyrimidinyl, pyrido[2,3-d]pyrimidinyl, pyrido[2,3-b]pyrazinyl, pyrido[3,4-b]pyrazinyl, pyrimido[5,4-d]pyrimidinyl, pyrazino[2,3-b]pyrazinyl, and pyrimido[4,5-d]pyrimidinyl. At least one hydrogen atom in the heteroaryl group may be substituted with one of the same substituents as described above in connection with the alkyl group.

The term "heteroarylalkyl" refers to alkyl substituted with heteroaryl.

The term "heteroaryloxy" includes an —O-heteroaryl moiety. At least one hydrogen atom in heteroaryloxy may be substituted with one of the same substituents as described above in connection with the alkyl group.

The term "heteraryloxyalkyl" refers to an alkyl group that is substituted with heteroaryloxy. At least one hydrogen atom in heteraryloxyalkyl may be substituted with the same substituent as described above in connection with the alkyl group.

As used herein, the term "carbocyclic" refers to saturated or partially unsaturated but non-aromatic monocyclic, bicyclic or tricyclic hydrocarbon groups. Exemplary monocyclic hydrocarbon groups include cyclopentyl, cyclopentenyl, cyclohexyl and cyclohexenyl. Exemplary bicyclic hydrocarbon groups include bornyl, decahydronaphthyl, bicyclo[2.1.1]hexyl, bicyclo[2.2.1]heptyl, bicyclo[2.2.1]heptenyl, and bicyclo[2.2.2]octyl. Exemplary tricyclic hydrocarbon groups include adamantyl. At least one hydrogen atom in carbocyclic may be substituted with one of the same substituents as described above in connection with the alkyl group.

The term "heterocyclic" refers to a ring containing 5-10 ring atoms including a hetero atom such as N, S, P, or O, and an example of heterocyclic is pyridyl. At least one hydrogen atom in heterocyclic may be substituted with one of the same substituents as described above in connection with the alkyl group.

The term "heterocyclicoxy" includes an —O-heterocyclyl, and at least one hydrogen atom in heterocyclicoxy may be substituted with one of the same substituents as described above in connection with the alkyl group.

The term "sulfonyl" includes R"-SO₂-, wherein R" is hydrogen, alkyl, aryl, heteroaryl, aryl-alkyl, heteroaryl-alkyl, alkoxy, aryloxy, cycloalkyl, or heterocyclyl.

The term "sulfamoyl" includes H ₂NS(O) ₂-, alkyl-NHS(O) ₂-, (alkyl) ₂NS(O) ₂-, aryl-NHS(O) ₂-, alkyl(aryl)-NS(O) ₂-, (aryl) ₂NS(O) ₂-, heteroaryl-NHS(O) ₂-, (aryl-alkyl)-NHS(O) ₂-, or (heteroaryl-alkyl)-NHS(O) ₂-. At least one hydrogen atom in sulfamoyl may be substituted with one of the same substituents as described above in connection with the alkyl group.

The term "amino" includes groups where a nitrogen atom is covalently bonded to at least one carbon or heteroatom. The term "amino" also includes -NH₂ and also includes substituted moieties.

The term also includes "alkyl amino" wherein the nitrogen is bound to at least one additional alkyl group. The term also includes "arylamino" and "diarylamino" groups wherein the nitrogen is bound to one or two independently selected aryl groups, respectively.

The term "alkylene", "alkenylene", "alkynylene", "arylene", and "heteroarylene" are defined as described above, except that "alkyl", "alkenyl", "alkynyl", "aryl", and "heteroaryl", which are mono-valent groups, are changed into divalent groups. At least one hydrogen atom in "alkylene", "alkenylene", "alkynylene", "arylene", and "heteroarylene" may be substituted with one of the same substituents as described above in connection with the alkyl group.

The fuel cell including the electrode with improved electrical conductive characteristics may have improved cell performance, in terms of current density. The fuel cell is suitable for use in high-temperature, non-humidified conditions.

Hereinafter, one or more embodiments of the present invention will be described in detail with reference to the following examples. These examples are not intended to limit the purpose and scope of the one or more embodiments of the present invention.

### Synthesis Example 1: Preparation of compound represented by Formula 9

4.4 moles of formaldehyde and 2.2 moles of 2-thiophene-methylamine were mixed with 120 ml of toluene and 40 ml of N, N-dimethylformamide, and a small catalytic amount of p-toluenesulfonic acid was added thereto. The mixture was reacted at about 80 °C for 1 hour, and 1 mol of bisphenol-S was added to the reacted mixture and reacted at about 120 °C for about 2 hours to obtain a crude product.

The crude product was washed twice with a 1 N NaOH aqueous solution and once with distilled water, and dried with magnesium sulfate. Subsequently, the resultant was filtered and then the solvent was removed therefrom. Then, the resultant was dried under vacuum to obtain a compound represented by Formula 9 below with a yield of 80%.

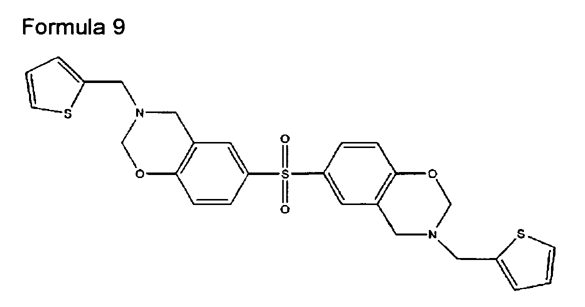

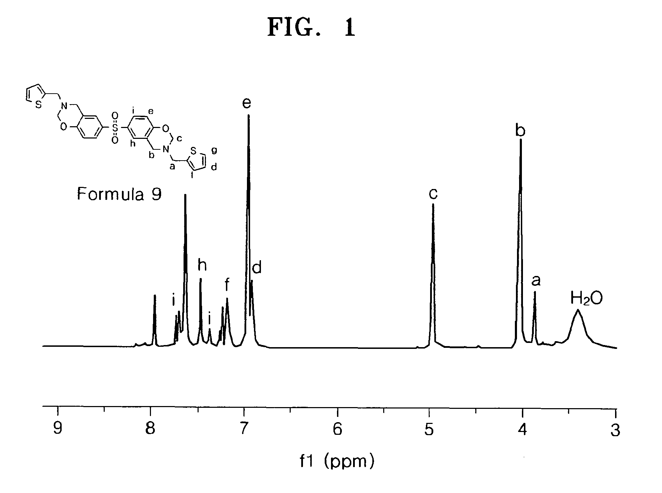

The structure of the obtained compound was identified using nuclear magnetic resonance (NMR) spectrometry. The result is shown in FIG. 1.

### Synthesis Example 2: Preparation of compound represented by Formula 19

A compound represented by Formula 19 below was prepared in the same manner as in Synthesis Example 1, except that 4-(thiophene-3-yl)aniline represented by Formula 18 below and phenol were used, instead of 2-thiophene-methylamine and bisphenol-S, respectively.

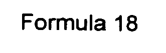

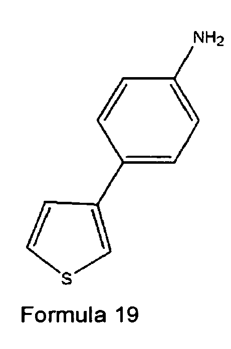

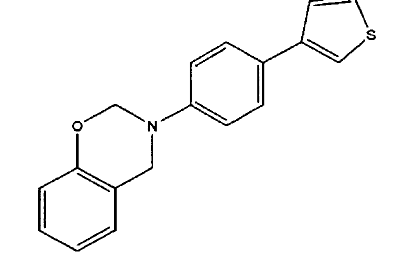

### Synthesis Example 3: Preparation of compound represented by Formula 10

A compound represented by Formula 10 below was prepared in the same manner as in Synthesis Example 1, except that 4-(thiophene-2-yl)aniline represented by Formula 20 below was used, instead of 2-thiophene-methylamine.

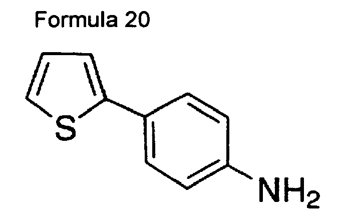

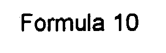

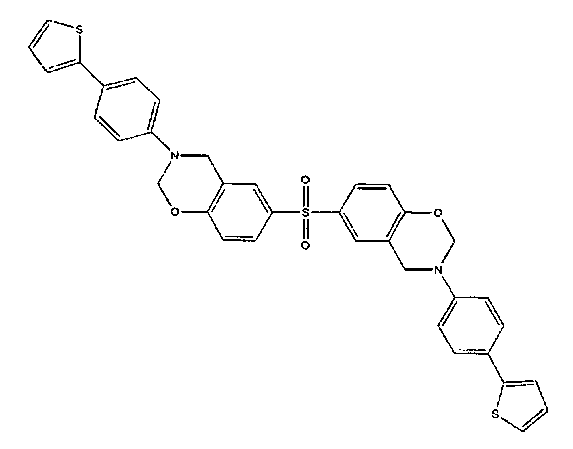

### Preparation Example 1: preparation of a composition

65 parts by weight of compound represented by Formula 9 prepared by Synthesis Example 1 and 35 parts by weight of compound represented by Formula 12 were mixed to obtain a composition.

### Example 1: Manufacture of electrodes for fuel cells and a fuel cell including the electrodes

1 g of carbon black and 4 g of ethylene glycol were mixed together and then that mixture was mixed with a mixed solution of 0.2 g of a compound represented by Formula 9 below in 0.8 g of N-methylpyrrolidone (NMP).

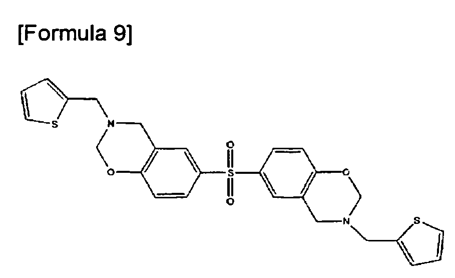

The resulting mixture was tape-cast on carbon paper treated with 5 wt% of polytetrafluoroethylene, and then thermally treated at about 250 °C to form a microporous layer on the carbon paper.

Meanwhile, 1 g of a Pt/Co catalyst (45 wt% of Pt and 5 wt% of Co on carbon) and 3 g of NMP acting as a solvent were put into a stirring vessel. The mixture was stirred using a high-speed stirrer to prepare a slurry.

Next, a solution of 5 wt% of a vinylidenefluoride-co-hexafluoropropylene copolymer in NMP was added to the mixture until the amount of the vinylidenefluoride-co-hexafluoropropylene copolymer in the mixture reached 0.026 g, followed by mixing for about 10 minutes to prepare a slurry for forming a cathode catalyst layer.

The slurry for forming a cathode catalyst layer was coated on the microporous layer, and the resultant was dried at room temperature for 1 hour, dried at about 80 °C for about 1 hour, dried at about 120 °C for about 30 minutes, and dried at about 150 °C for about 15 minutes to manufacture a cathode. The amount of Pt loaded in the cathode was 1.5 mg/cm².

An anode was manufactured as follows. 0.5 g of a Pt/Ru catalyst (30 wt% of Pt and 15 wt% of Ru supported on carbon) and 6 g of NMP acting as a solvent were put into a stirring vessel, and stirred using a high-speed stirrer for about two minutes. Subsequently, a solution of 0.05 g of polyvinylidenefluoride in 1 g of NMP was added to the mixture, followed by further stirring for about two minutes to prepare a slurry for forming an anode catalyst layer. The slurry for forming an anode catalyst layer was coated on carbon paper that was coated with the microporous layer, using a bar coater to manufacture an anode. The amount of Pt loaded in the anode was 0.9 mg/cm².

Meanwhile, 65 parts by weight of a compound represented by Formula 62 below and 35 parts by weight of polybenzimidazole (m-PBI) represented by Formula 12 below were blended, and the blend was cured at a temperature of about 80 °C to about 220 °C.

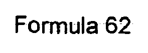

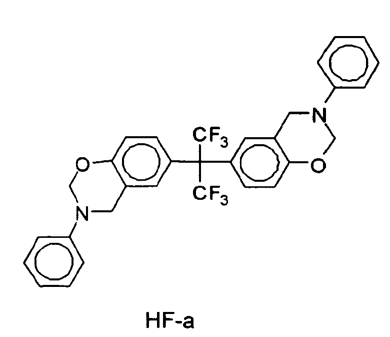

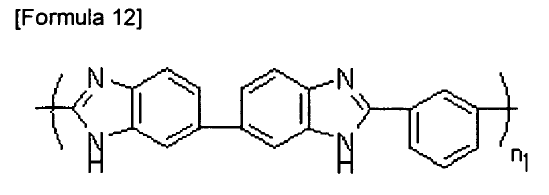

Subsequently, the resultant was impregnated with 85 wt% of phosphoric acid at about 80 °C for about 4 hours or longer to form an electrolyte membrane. The amount of the phosphoric acid was about 300 parts by weight based on 100 parts by total weight of the electrolyte membrane.

The electrolyte membrane was disposed between the cathode and the anode to manufacture a membrane-electrode assembly (MEA).

To prevent gas permeation between the cathode and the anode, a PTFE membrane main-gasket having a thickness of 200 µm and a PTFE membrane sub-gasket having a thickness of 20 µm were joined and disposed between each of the anode and cathode, and the electrolyte membrane. The pressure applied to the MEAs was adjusted using a torque wrench, and was stepwise increased using 1, 2, and 3 N-m Torque wrenches.

Electricity was generated by supplying hydrogen to the anode (flow rate: 100 ccm) and air to the cathode (flow rate: 250 ccm), at 150 °C under non- humid conditions on the electrolyte membrane, and characteristics of the fuel cell were measured. Based on the fact that performance of fuel cells using a phosphoric acid-doped electrolyte is improved with time, the fuel cell was activated until the operating voltage reached a peak voltage, and then the characteristics of the fuel cell were evaluated. The area of each of the cathode and the anode was set to 7.84 cm² (=2.8 cmx2.8 cm). The cathode was about 490 µm thick and the anode was about 390 µm thick.

### Example 2: Manufacture of electrodes for fuel cells and a fuel cell including the electrodes

1 g of carbon black and 4 g of ethylene glycol were mixed together and then that mixture was mixed with a mixed solution of 0.2 g of polytetrafluoroethylene in 0.8 g of N-methylpyrrolidone (NMP).

The resulting mixture was tape-cast on carbon paper treated with 5 wt% of polytetrafluoroethyelene, and then thermally treated at about 250 °C to form a microporous layer on the carbon paper.

Meanwhile, 1 g of a Pt/Co catalyst (45 wt% of Pt and 5 wt% of Co on carbon) and 3 g of NMP as a solvent were put into a stirring vessel. The mixture was stirred using a high-speed stirrer to prepare a slurry.

Next, a 20 wt% solution of the compound of Formula 9 in NMP was added to the mixture until the amount of the compound of Formula 9 in the mixture reached 0.025 g, followed by mixing for about 10 minutes to prepare a slurry for forming a cathode catalyst layer.

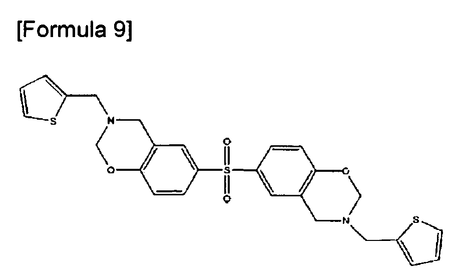

The slurry for forming the cathode catalyst layer was coated on the microporous layer, and the resultant was dried at room temperature for about 1 hour, dried at about 80 °C for about 1 hour, dried at about 120 °C for about 30 minutes, and dried at about 150 °C for about 15 minutes to manufacture a cathode. The amount of Pt loaded in the cathode was 1.5 mg/cm².

An anode was manufactured as follows. 2 g of a Pt/Ru catalyst, including 30 wt% of Pt and 15 wt% of Ru, supported on carbon, and 9 g of NMP as a solvent were put into a stirring vessel, and the mixture was stirred using a high-speed stirrer for about 2 minutes.

Subsequently, a solution of 0.05 g of polyvinylidenefluoride in 1 g of NMP was added to the mixture, followed by further stirring for about two minutes to prepare a slurry for forming an anode catalyst layer. The slurry for forming the anode catalyst layer was coated on carbon paper that was coated with the microporous layer, using a bar coater to manufacture an anode. The amount of Pt loaded in the anode was 0.9 mg/cm².

Meanwhile, 65 parts by weight of a compound represented by Formula 62 below and 35 parts by weight of polybenzimidazole (m-PBI) represented by Formula 12 below were blended, and the blend was cured at a temperature of about 80 °C to about 220 °C.

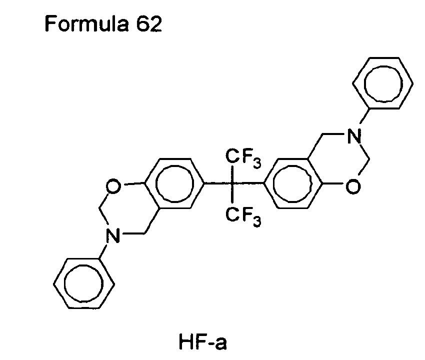

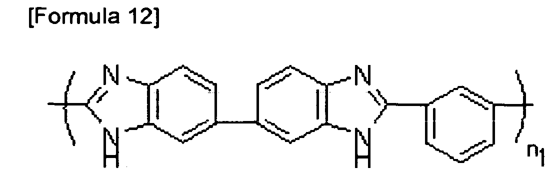

Subsequently, the resultant was impregnated with 85 wt% of phosphoric acid at about 80 °C for about 4 hours or longer to form an electrolyte membrane. The amount of the phosphoric acid was about 500 parts by weight based on 100 parts by weight of the electrolyte membrane.

The electrolyte membrane was disposed between the cathode and the anode to manufacture a membrane-electrode assembly (MEA). The cathode and the anode were not impregnated with phosphoric acid.

To prevent gas permeation between the cathode and the anode, a PTFE membrane main-gasket having a thickness of 200 µm and a PTFE membrane sub-gasket having a thickness of 20 µm were joined and disposed between each of the anode and cathode, and the electrolyte membrane. The pressure applied to the MEAs was adjusted using a torque wrench, and was stepwise increased using 1, 2, and 3 N-m Torque wrenches.

Electricity was generated by supplying hydrogen to the anode (flow rate: 100 ccm) and air to the cathode (flow rate: 250 ccm), at 150 °C under non-humid conditions on the electrolyte membrane, and characteristics of the fuel cell were measured. Based on the fact that performance of fuel cells using a phosphoric acid-doped electrolyte is improved with time, the fuel cell was activated until the operating voltage reached a peak voltage, and then the characteristics of the fuel cell were evaluated. The area of each of the cathode and the anode was set to 7.84 cm² (=2.8 cmx2.8 cm). The cathode was about 390 µm thick and the anode was about 390 µm thick.

### Example 3: Manufacture of electrodes for fuel cells and a fuel cell including the electrodes

Electrodes for fuel cells and a fuel cell were manufactured in the same manner as in Example 1, except that the amount of the compound of Formula 9 was varied to 0.3 g when forming the microporous layer.

### Example 4: Manufacture of electrodes for fuel cells and a fuel cell including the electrodes

Electrodes for fuel cells and a fuel cell were manufactured in the same manner as in Example 1, except that the amount of the compound of Formula 9 was varied to 0.4 g when forming the microporous layer.

### Example 5: Manufacture of electrodes for fuel cells and a fuel cell including the electrodes

Electrodes for fuel cells and a fuel cell were manufactured in the same manner as in Example 1, except that, instead of the common carbon paper treated with 5 wt% of polytetrafluoroethylene, a gas diffusion layer prepared by the following process was used as the carbon paper when forming the cathode and anode.

A compound represented by Formula 9 below and N-methylpyrrolidone (NMP) were mixed to obtain 8 wt% of the compound represented by Formula 9 in NMP solution.

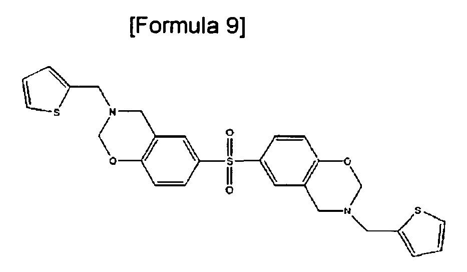

A carbon paper (Toray 060 plain) was dip-coated by 8 wt% of the compound represented by Formula 9 in NMP solution, and then the resulting carbon paper was thermally treated at about 250 °C to form a gas diffusion layer.

1 g of carbon black and 4 g of ethylene glycol were mixed together and then were mixed with a mixed solution of 0.2 g of the compound represented by Formula 9 in 0.8 g of N-methylpyrrolidone (NMP).

The resulting mixture was tape-cast on the gas diffusion layer and then thermally treated at about 250 °C to form a microporous layer.

### Comparative Example 1: Manufacture of electrodes for fuel cells and a fuel cell including the electrodes

Electrodes for fuel cells and a fuel cell were manufactured in the same manner as in Example 1, except that 0.3 g of polytetrafluoroethylene (PTFE) was used, instead of 0.2 g of the compound of Formula 9, when forming the microporous layer.

### Comparative Example 2: Manufacture of electrodes for fuel cells and a fuel cell including the electrodes

Electrodes for fuel cells and a fuel cell were manufactured in the same manner as in Example 2, except that 0.3 g of polytetrafluoroethylene (PTFE) was used, instead of 0.025 g of the compound of Formula 9, when forming the cathode catalyst layer.

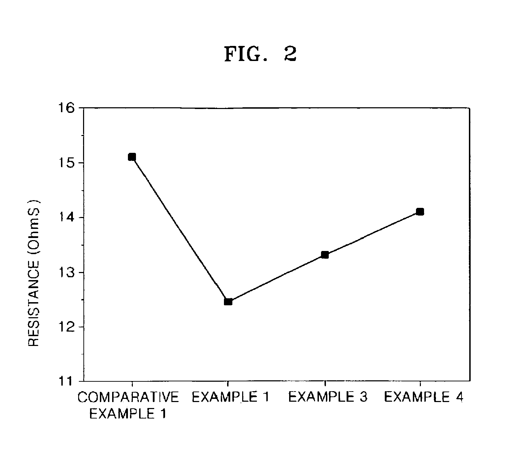

Conductivities of the microporous layers formed in Examples 1, 3, 4 and Comparative Example 1 were measured. The results are shown in FIG. 2. Referring to FIG. 2, the microporous layers of Examples 1, 3 and 4 are found to have improved conductivities, as compared to that of Comparative Example 1.

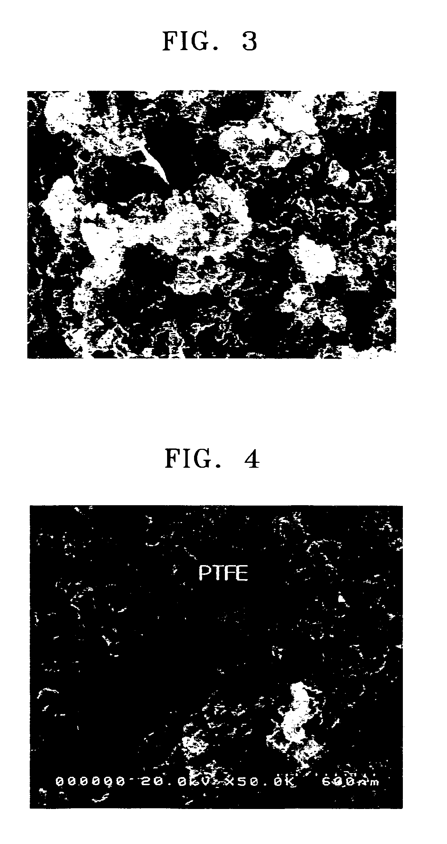

The microporous layers of Example 3 and Comparative Example 1 were analyzed using scanning electron microscopy (SEM). The results are shown in FIGS. 3 and 4, respectively. Referring to FIGS. 3 and 4, in the microporous layer of Example 3 components appear uniformly dispersed. Meanwhile, in the microporous layer of Comparative Example 1, PTFE appears agglomerated.

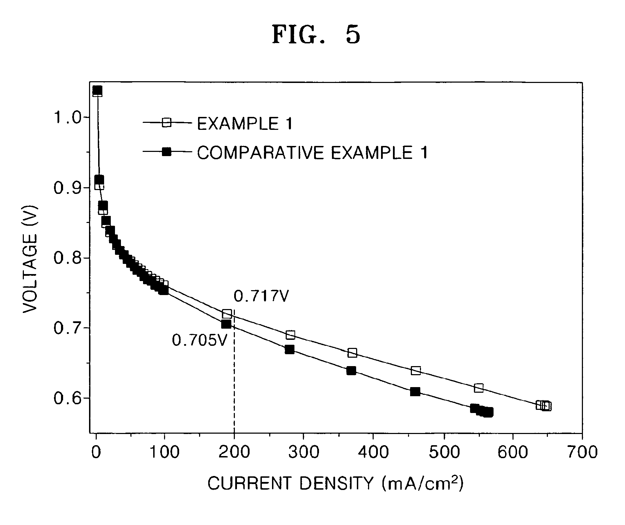

Changes in cell voltages of the fuel cells manufactured in Example 1 and Comparative Example 1 with respect to current density were measured. The results are shown in FIG. 5. Referring to FIG. 5, the fuel cell of Example 1 has improved cell voltage characteristics, as compared to the fuel cell of Comparative Example 1.

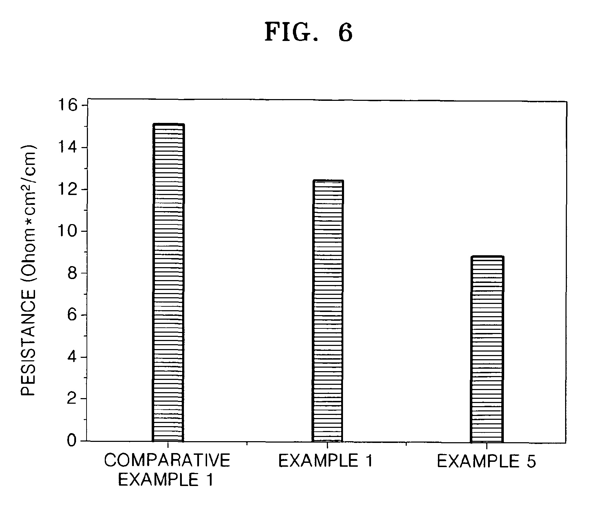

Changes in conductivities of the microporous layers formed in Examples 1, 5 and Comparative Example 1 were measured. The results are shown in FIG. 6. Referring to FIG. 6, the microporous layers of Examples 1 and 5 have improved conductivities, as compared to the microporous layer of Comparative Example 1.

As described above, according to the one or more of the above embodiments of the present invention, a composition including a conductive functional group and a polymer that is a polymerization product of the composition may have good resistance to chemicals, heat, and acid. An electrode for fuel cells that includes the same may have improved electrical conductivity. A fuel cell with improved current density may be manufactured using the electrode.

It should be understood that the exemplary embodiments described herein should be considered in a descriptive sense only and not for purposes of limitation. Descriptions of features or aspects within each embodiment should typically be considered as available for other similar features or aspects in other embodiments.

Although a few embodiments of the present invention have been shown and described, it would be appreciated by those skilled in the art that changes may be made in this embodiment without departing from the principles of the invention, the scope of which is defined in the claims.

## Claims

1. A composition comprising at least one of a compound represented by Formula 1 below and a compound represented by Formula 2 below: 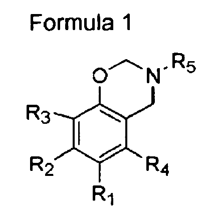 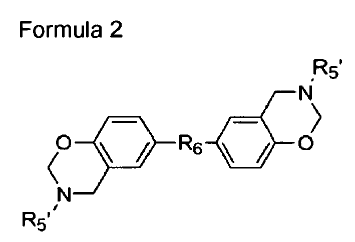
wherein, in Formulae 1 and 2, R₁ to R₄ are each independently a hydrogen atom, a substituted or unsubstituted C₁-C₂₀ alkyl group, a substituted or unsubstituted C₁-C₂₀ alkoxy group, a substituted or unsubstituted C₂-C₂₀ alkenyl group, a substituted or unsubstituted C₂-C₂₀ alkynyl group, a substituted or unsubstituted C₆-C₂₀aryl group, a substituted or unsubstituted C₆-C₂₀ aryloxy group, a substituted or unsubstituted C₂-C₂₀ heteroaryl group, a substituted or unsubstituted C₂-C₂₀ heteroaryloxy group, a substituted or unsubstituted C₄-C₂₀ carbocyclic group, a substituted or unsubstituted C₄-C₂₀ carbocyclic oxy group, a substituted or unsubstituted C₂-C₂₀ heterocyclic group, a halogen atom, a hydroxyl group, or a cyano group;
R₆ is selected from the group consisting of a substituted or unsubstituted C₁-C₂₀ alkylene group, a substituted or unsubstituted C₂-C₂₀ alkenylene group, a substituted or unsubstituted C₂-C₂₀ alkynylene group, a substituted or unsubstituted C₆-C₂₀ arylene group, a substituted or unsubstituted C₂-C₂₀ heteroarylene group, -C(=O)-, and -SO₂-; and
R₅ and R_{5'} are each independently selected from the group consisting of a group represented by Formula 3 below, a group represented by Formula 4 below, and -S≡N, 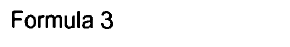 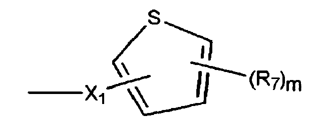 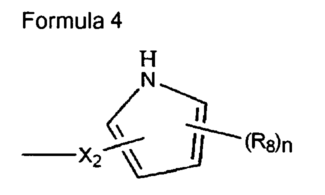
wherein, in Formulae 3 and 4, X₁ and X₂ are each independently a substituted or unsubstituted C₁-C₂₀ alkylene group, a substituted or unsubstituted C₆-C₂₀ arylene group, or a substituted or unsubstituted C₂-C₂₀ heteroarylene group;
R₇ and R₈ are each independently a hydrogen atom, a substituted or unsubstituted C₁-C₂₀ alkyl group, a substituted or unsubstituted C₆-C₂₀ aryl group, a substituted or unsubstituted C2-C20 heteroaryl group, a substituted or unsubstituted C₄-C₂₀ carbocyclic group, a substituted or unsubstituted C₂-C₂₀ heterocyclic group, a halogen atom, a hydroxyl group, an amino group, or a cyano group; and
m and n are each independently an integer from 1 to 3.

2. The composition of claim 1, wherein R₅ and R₅' are each independently one of the groups represented by Formulae 5A - 5C below: 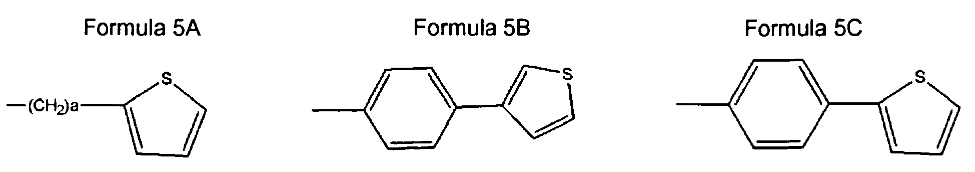 wherein, in Formula 5A, a is an integer from 1 to 5.

3. The composition of claim 1, wherein the compound of Formula 2 is selected from the compounds represented by Formulae 6 to 8 below: 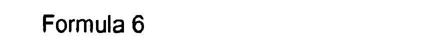 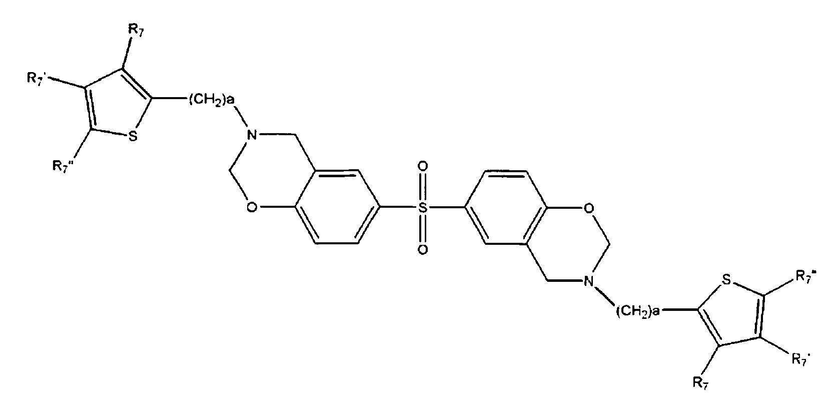 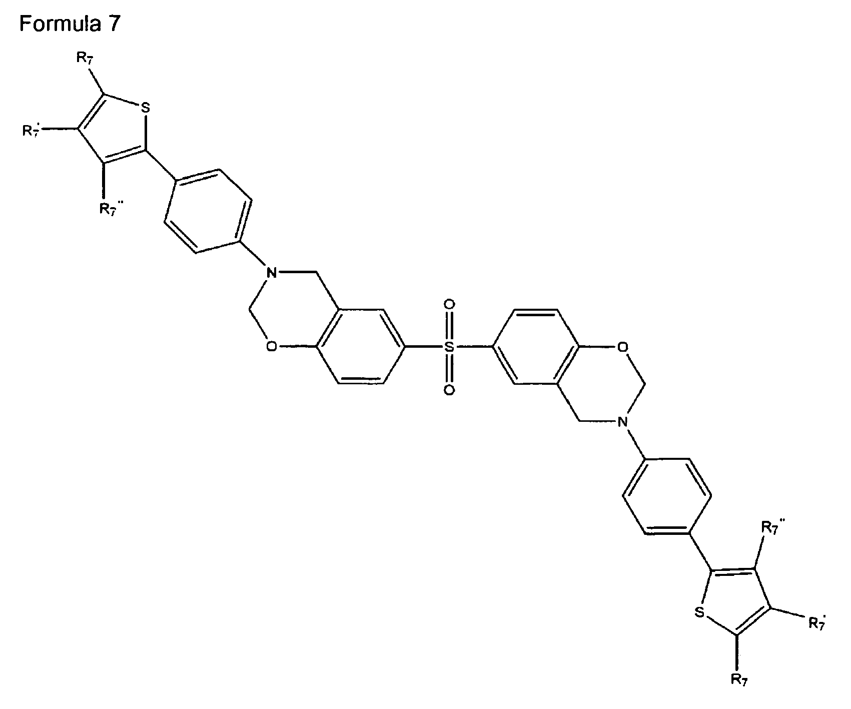 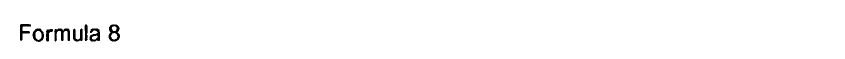 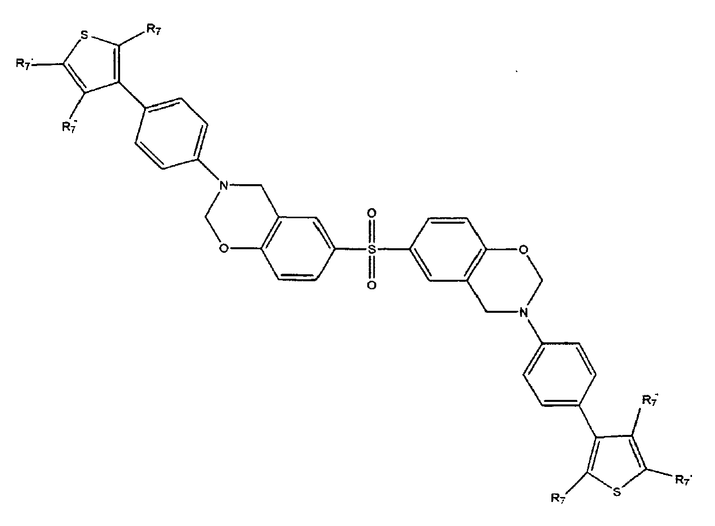
wherein, in Formulae 6 to 8, R₇, R₇' and R₇" are each independently a hydrogen atom, a C₁-C₂₀ alkyl group, a C₆-C₂₀ aryl group, a C₂-C₂₀ heteroaryl group, a C₂-C₂₀ heteroaryloxy group, a C₄-C₂₀ carbocyclic group, a C₂-C₂₀ heterocyclic group, a halogen atom, a hydroxyl group, or a cyano group; and
a is an integer from 1 to 5.

4. The composition of claim 1, wherein the compound of Formula 2 is selected from the compounds represented by Formulae 9 to 11, and 19 below:

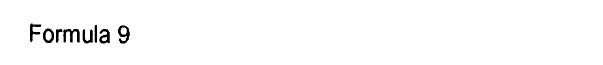

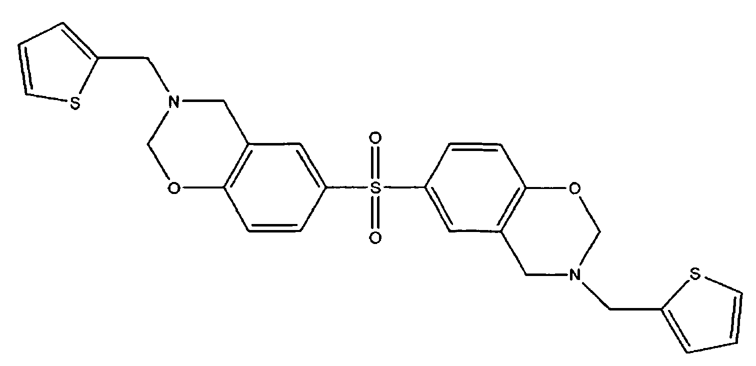

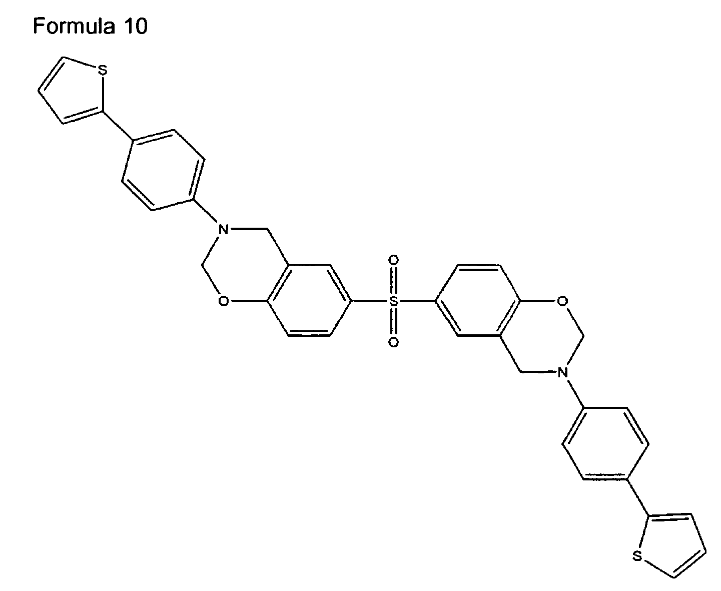

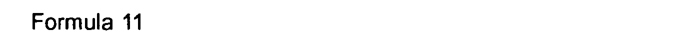

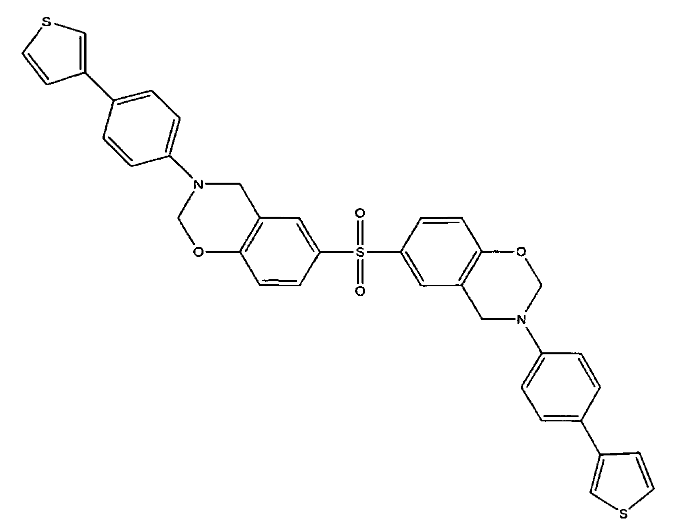

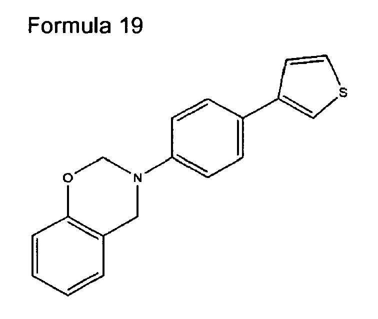

5. The composition of any of claims 1-4, further comprising a cross-linkable compound.

6. The composition of claim 5, wherein the cross-linkable compound is at least one material selected from the group consisting of a polyazole-based material, polyimide and polyoxazole.

7. The composition of claim 6, wherein the cross-linkable compound comprises at least one compound selected from among the compounds represented by Formulae 12 to 14 below: 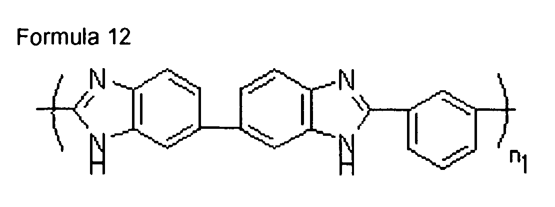
wherein, in Formula 12, n₁ is an integer of 10 or greater; 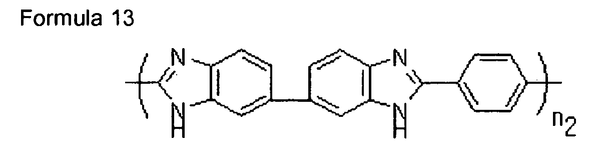
wherein, in Formula 13, n₂ is an integer of 10 or greater, 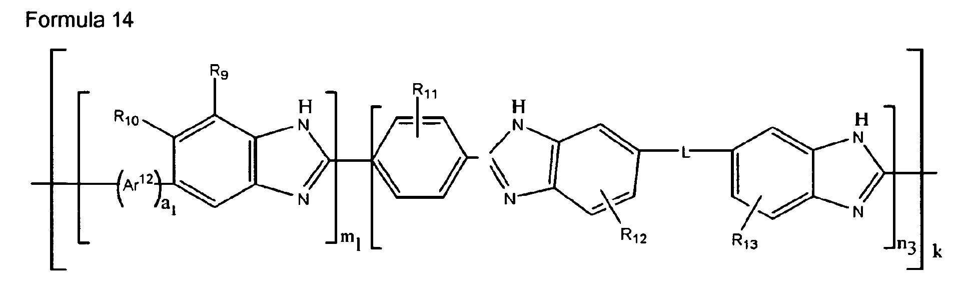
wherein, in Formula 14, R₉ and R₁₀ are each independently a hydrogen atom, an unsubstituted or substituted C₁-C₂₀ alkyl group, an unsubstituted or substituted C₁-C₂₀ alkoxy group, an unsubstituted or substituted C₆-C₂₀ aryl group, an unsubstituted or substituted C₆-C₂₀ aryloxy group, an unsubstituted or substituted C₃-C₂₀ heteroaryl group, or an unsubstituted or substituted C₃-C₂₀ heteroaryloxy group, wherein
R₉ and R₁₀ may be linked to form a C₄-C₂₀ carbocyclic group or a C₃-C₂₀heterocyclic group;
Ar¹² is a substituted or unsubstituted C₆-C₂₀ arylene group or a substituted or unsubstituted C₃-C₂₀ heteroarylene group;
R₁₁ to R₁₃ are each independently a mono- or a multi-substituted substituent selected from the group consisting of a hydrogen atom, a substituted or unsubstituted C₁-C₂₀ alkyl group, a substituted or unsubstituted C₁-C₂₀ alkoxy group, a substituted or unsubstituted C₆-C₂₀ aryl group, a substituted or unsubstituted C₆-C₂₀ aryloxy group, a substituted or unsubstituted C₃-C₂₀ heteroaryl group, or a substituted or unsubstituted C₃-C₂₀ heteroaryloxy group;
L represents a linker;
m₁ is from 0.01 to 1;
a₁ is 0 or 1;
n₃ is a number from 0 to 0.99; and
k is a number from 10 to 250.

8. A polymer that is a polymerization product of the composition of any of claims 1-7.

9. A method of preparing a composition according to any of claims 1-4, the method comprising thermally treating a mixture of a phenol compound (A) below, formaldehyde, an amine compound (B) below, an aprotic polar solvent and an organic solvent to obtain the at least one of the compounds of Formulae 1 and 2: 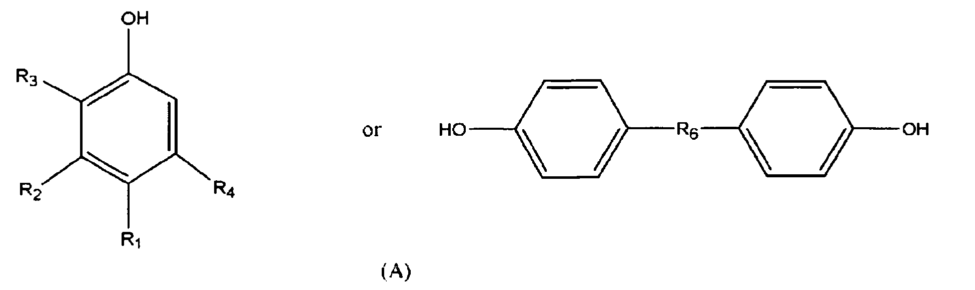
R₅NH₂ or R₅'NH₂ (B) 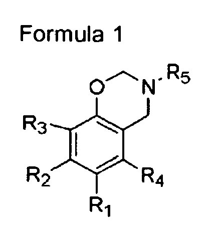 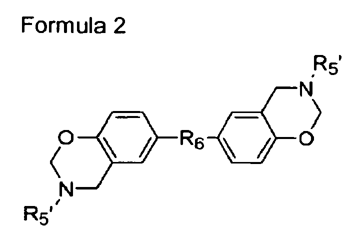
wherein, R₁ to R₄ in the formulae of the phenol compound (A) and R₅, R₅' in the amine compound (B), are the same as R₁ to R₄ and R₅, R₅' in Formulae 1 and 2.

10. The method of claim 9, wherein the aprotic polar solvent is at least one solvent selected from the group consisting of N, N'-dimethylformamide, N, N'-dimethylacetamide, N-methylpyrrolidone, tetramethylenesulfone, 1,2-dimethyl-2-imidazolidinone, and N-methylformamide, and/or.
wherein the organic solvent comprises at least one solvent selected from among 1,4-dioxane, chloroform, dichloromethane, tetrahydrofuran (THF), a benzene-based solvent preferably selected from the group consisting of benzene, toluene and xylene, and a mixture thereof

11. The method of claim 9 or 10, wherein at least one catalyst selected from among p-toluenesulfonic acid, phosphorus pentachloride (PCl₅), and phosphoryl chloride (POCl₃) is further added when the phenol compound (A), the formaldehyde, and the amine compound (B) are mixed together.

12. An electrode of a fuel cell, the electrode comprising a composition according to any of claims 1-7 or a polymer according to claim 8.

13. The electrode of claim 12, wherein the electrode further comprises at least one of a gas diffusion layer and a microporous layer and a catalyst layer; and
the at least one of the gas diffusion layer, the microporous layer and the catalyst layer comprises the polymer.

14. An electrolyte membrane of a fuel cell, the electrolyte membrane comprising a polymer according to claim 8.

15. A fuel cell comprising:
a cathode;
an anode; and
an electrolyte membrane between the cathode and the anode,
wherein at least one of the cathode, the anode and the electrolyte membrane comprises a composition according to claims 1-7 or a polymer according to claim 8.

## Patentansprüche

1. Zusammensetzung, die mindestens eine von einer Verbindung, die durch Formel 1 nachstehend dargestellt ist, und einer Verbindung, die durch Formel 2 nachstehend dargestellt ist, umfasst: 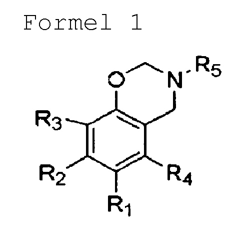 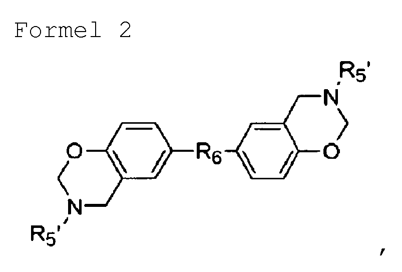
worin in den Formeln 1 und 2 R₁ bis R₄ jeweils unabhängig Folgendes sind: ein Wasserstoffatom, eine substituierte oder unsubstituierte C₁-C₂₀-Alkylgruppe, eine substituierte oder unsubstituierte C₁-C₂₀-Alkoxygruppe, eine substituierte oder unsubstituierte C₂-C₂₀-Alkenylgruppe, eine substituierte oder unsubstituierte C₂-C₂₀-Alkinylgruppe, eine substituierte oder unsubstituierte C₆-C₂₀-Arylgruppe, eine substituierte oder unsubstituierte C₆-C₂₀-Aryloxygruppe, eine substituierte oder unsubstituierte C₂-C₂₀-Heteroarylgruppe, eine substituierte oder unsubstituierte C₂-C₂₀-Heteroaryloxygruppe, eine substituierte oder unsubstituierte carbocyclische C₄-C₂₀-Gruppe, eine substituierte oder unsubstituierte carbocyclische C₄-C₂₀-Oxygruppe, eine substituierte oder unsubstituierte heterocyclische C₂-C₂₀-Gruppe, ein Halogenatom, eine Hydroxylgruppe oder eine Cyanogruppe;
R₆ aus der Gruppe ausgewählt ist, die aus Folgenden besteht: einer substituierten oder unsubstituierten C₁-C₂₀-Alkylengruppe, einer substituierten oder unsubstituierten C₂-C₂₀-Alkenylengruppe, einer substituierten oder unsubstituierten C₂-C₂₀-Alkinylengruppe, einer substituierten oder unsubstituierten C₆-C₂₀-Arylengruppe, einer substituierten oder unsubstituierten C₂-C₂₀-Heteroarylengruppe, -C(=O)- und -SO₂-; und
R₅ und R₅' jeweils unabhängig aus der Gruppe ausgewählt sind, die aus einer Gruppe, die durch Formel 3 nachstehend dargestellt ist, einer Gruppe, die durch Formel 4 nachstehend dargestellt ist, und -S=N besteht: 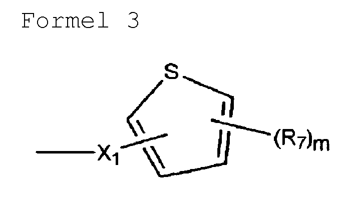 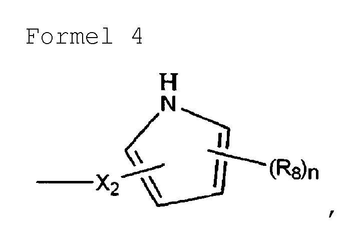
worin in den Formeln 3 und 4 X₁ und X₂ jeweils unabhängig Folgendes sind: eine substituierte oder unsubstituierte C₁-C₂₀-Alkylengruppe, eine substituierte oder unsubstituierte C₆-C₂₀-Arylengruppe oder eine substituierte oder unsubstituierte C₂-C₂₀-Heteroarylengruppe;
R₇ und R₈ jeweils unabhängig Folgendes sind: ein Wasserstoffatom, eine substituierte oder unsubstituierte C₁-C₂₀-Alkylgruppe, eine substituierte oder unsubstituierte C₆-C₂₀-Arylgruppe, eine substituierte oder unsubstituierte C₂-C₂₀-Heteroarylgruppe, eine substituierte oder unsubstituierte carbocyclische C₄-C₂₀-Gruppe, eine substituierte oder unsubstituierte heterocyclische C₂-C₂₀-Gruppe, ein Halogenatom, eine Hydroxylgruppe, eine Aminogruppe oder eine Cyanogruppe; und
m und n jeweils unabhängig eine ganze Zahl von 1 bis 3 sind.

2. Zusammensetzung nach Anspruch 1, worin R₅ und R₅' jeweils unabhängig eine der Gruppen sind, die durch die Formeln 5A-5C nachstehend dargestellt sind: 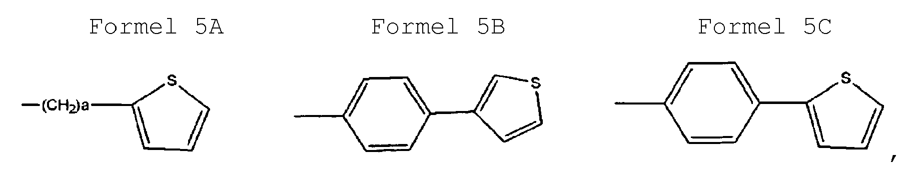 worin in Formel 5A a eine ganze Zahl von 1 bis 5 ist.

3. Zusammensetzung nach Anspruch 1, worin die Verbindung von Formel 2 aus den Verbindungen ausgewählt ist, die durch die Formeln 6 bis 8 nachstehend dargestellt sind: 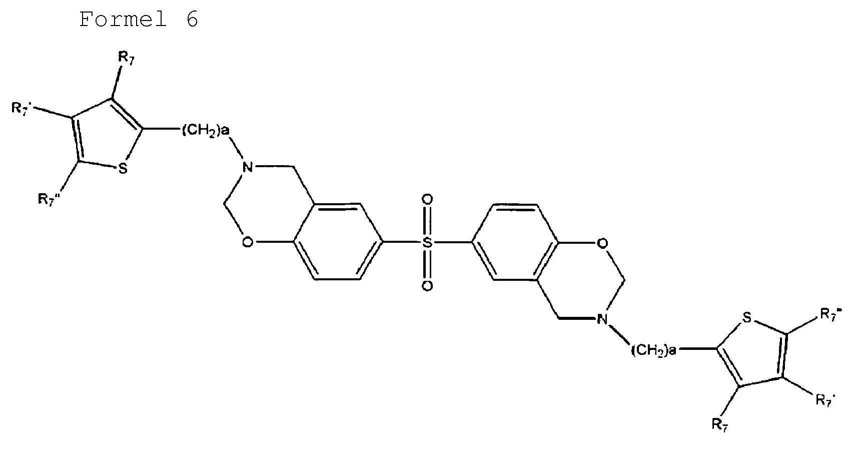 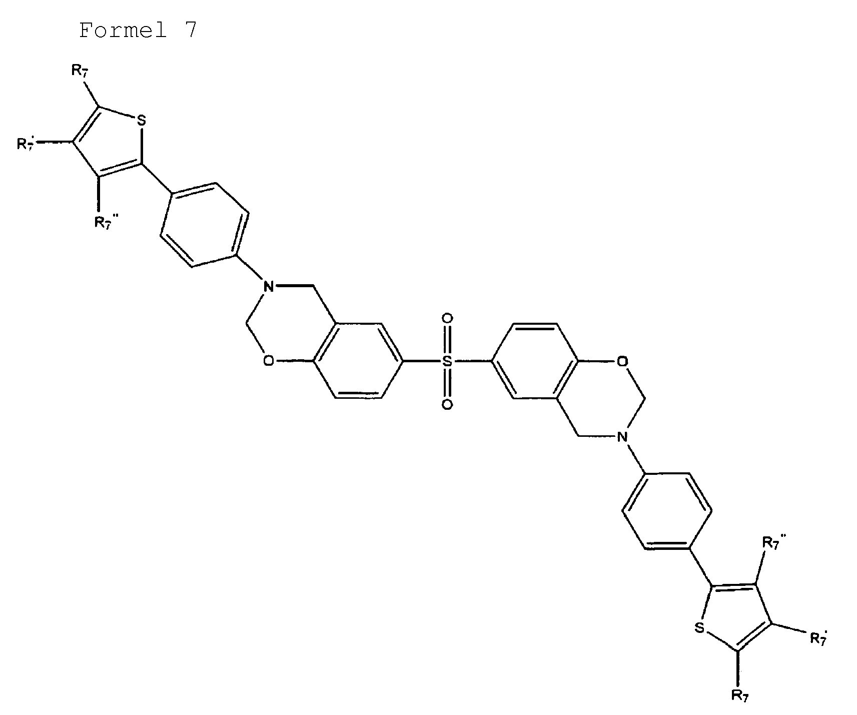 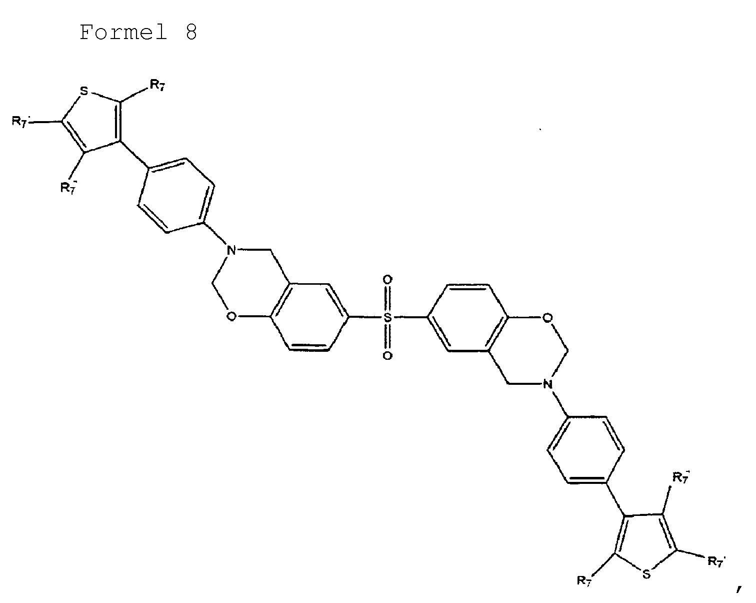
worin in den Formeln 6 bis 8 R₇, R₇' und R₇" jeweils unabhängig Folgendes sind: ein Wasserstoffatom, eine C₁-C₂₀-Alkylgruppe, eine C₆-C₂₀-Arylgruppe, eine C₂-C₂₀-Heteroarylgruppe, eine C₂-C₂₀-Heteroaryloxygruppe, eine carbocyclische C₄-C₂₀-Gruppe, eine heterocyclische C₂-C₂₀-Gruppe, ein Halogenatom, eine Hydroxylgruppe oder eine Cyanogruppe; und
a eine ganze Zahl von 1 bis 5 ist.

4. Zusammensetzung nach Anspruch 1, worin die Verbindung von Formel 2 aus den Verbindungen ausgewählt ist, die durch die Formeln 9 bis 11 und 19 nachstehend dargestellt sind:

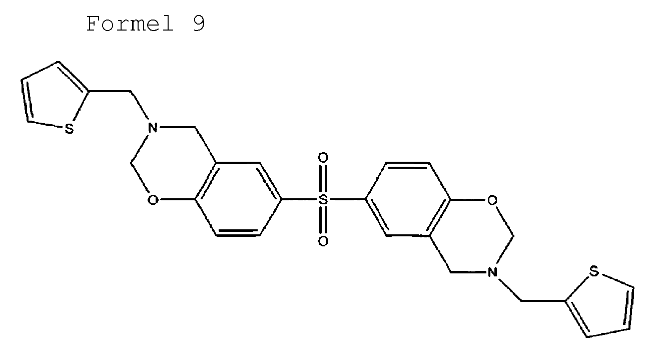

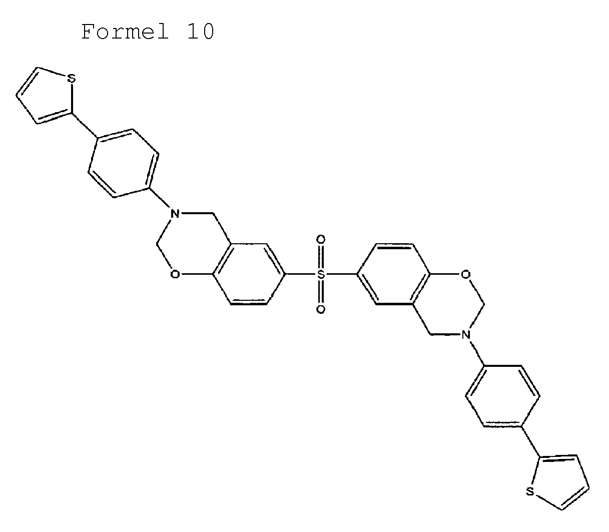

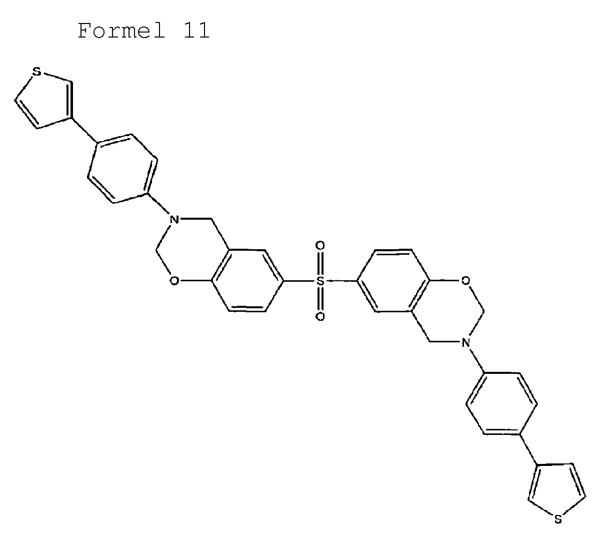

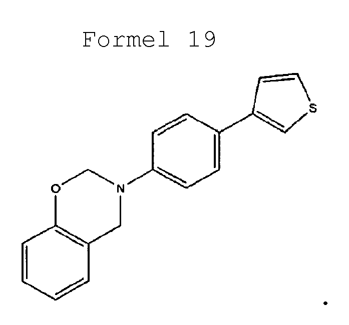

5. Zusammensetzung nach einem der Ansprüche 1-4, die weiter eine vernetzbare Verbindung umfasst.

6. Zusammensetzung nach Anspruch 5, worin die vernetzbare Verbindung mindestens ein Material ist, das aus der Gruppe ausgewählt ist, die aus einem Polyazol-basierten Material, Polyimid und Polyoxazol besteht.

7. Zusammensetzung nach Anspruch 6, worin die vernetzbare Verbindung mindestens eine Verbindung umfasst, die aus den Verbindungen ausgewählt ist, die durch die Formeln 12 bis 14 nachstehend dargestellt sind: 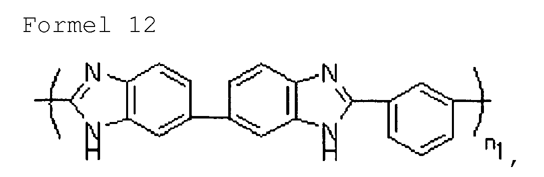
worin in Formel 12 n₁ eine ganze Zahl von 10 oder größer ist; 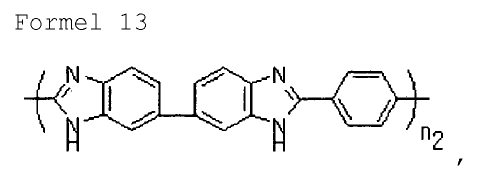
worin in Formel 13 n₂ eine ganze Zahl von 10 oder größer ist; 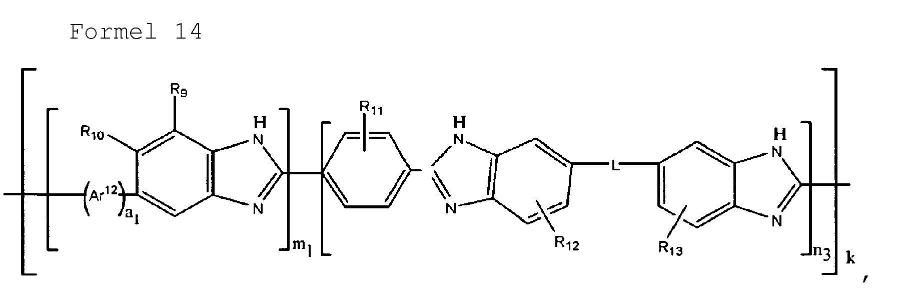
worin in Formel 14 R₉ und R₁₀ jeweils unabhängig Folgendes sind: ein Wasserstoffatom, eine unsubstituierte oder substituierte C₁-C₂₀-Alkylgruppe, eine unsubstituierte oder substituierte C₁-C₂₀-Alkoxygruppe, eine unsubstituierte oder substituierte C₆-C₂₀-Arylgruppe, eine unsubstituierte oder substituierte C₆-C₂₀-Aryloxygruppe, eine unsubstituierte oder substituierte C₃-C₂₀-Heteroarylgruppe oder eine unsubstituierte oder substituierte C₃-C₂₀-Heteroaryloxygruppe, worin R₉ und R₁₀ verbunden sein können, um eine carbocyclische C₄-C₂₀-Gruppe oder eine heterocyclische C₃-C₂₀-Gruppe zu bilden;
Ar¹² eine substituierte oder unsubstituierte C₆-C₂₀-Arylengruppe oder eine substituierte oder unsubstituierte C₃-C₂₀-Heteroarylengruppe ist;
R₁₁ bis R₁₃ jeweils unabhängig ein einfach oder ein mehrfach substituierter Substituent sind, der aus der Gruppe ausgewählt ist, die aus Folgenden besteht: einem Wasserstoffatom, einer substituierten oder unsubstituierten C₁-C₂₀-Alkylgruppe, einer substituierten oder unsubstituierten C₁-C₂₀-Alkoxygruppe, einer substituierten oder unsubstituierten C₆-C₂₀-Arylgruppe, einer substituierten oder unsubstituierten C₆-C₂₀-Aryloxygruppe, einer substituierten oder unsubstituierten C₃-C₂₀-Heteroarylgruppe oder einer substituierten oder unsubstituierten C₃-C₂₀-Heteroaryloxygruppe;
L einen Linker darstellt;
m₁ von 0,01 bis 1 ist;
a₁ 0 oder 1 ist;
n₃ eine Zahl von 0 bis 0,99 ist; und
k eine Zahl von 10 bis 250 ist.

8. Polymer, das ein Polymerisationsprodukt der Zusammensetzung nach einem der Ansprüche 1-7 ist.

9. Verfahren zur Herstellung einer Zusammensetzung nach einem der Ansprüche 1-4, wobei das Verfahren das thermische Behandeln einer Mischung aus einer Phenolverbindung (A) nachstehend, Formaldehyd, einer Aminverbindung (B) nachstehend, einem aprotischen polaren Lösungsmittel und einem organischen Lösungsmittel umfasst, um die mindestens eine der Verbindungen der Formeln 1 und 2 zu erhalten: 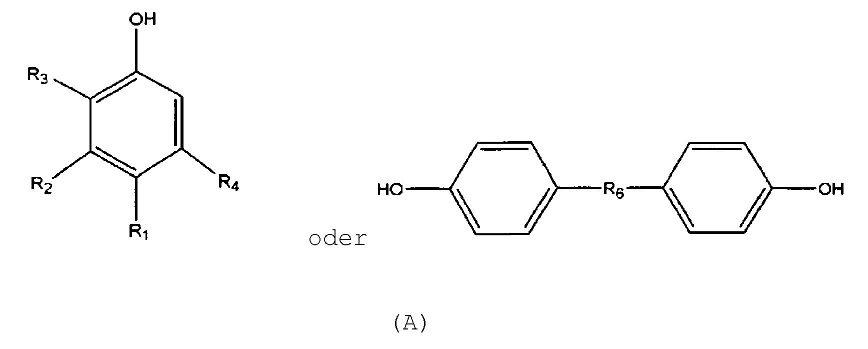
R₅NH₂ oder R₅'NH₂ (B) 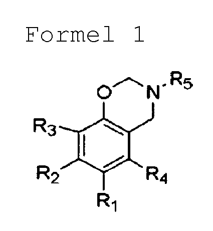 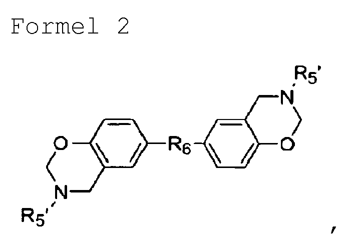
worin R₁ bis R₄ in den Formeln der Phenolverbindung (A) und R₅, R₅" in der Aminverbindung (B) gleich R₁ bis R₄ und R₅, R₅' in den Formeln 1 und 2 sind.

10. Verfahren nach Anspruch 9, worin das aprotische polare Lösungsmittel mindestens ein Lösungsmittel ist, das aus der Gruppe ausgewählt ist, die aus Folgenden besteht: N,N'-Dimethylformamid, N,N'-Dimethylacetamid, N-Methylpyrrolidon, Tetramethylensulfon, 1,2-Dimethyl-2-imidazolidinon und N-Methylformamid, und/oder
worin das organische Lösungsmittel mindestens ein Lösungsmittel umfasst, das aus Folgenden ausgewählt ist: 1,4-Dioxan, Chloroform, Dichlormethan, Tetrahydrofuran (THF), einem Benzol-basierten Lösungsmittel, das bevorzugt aus der Gruppe ausgewählt ist, die aus Benzol, Toluol und Xylol besteht, und einer Mischung davon.

11. Verfahren nach Anspruch 9 oder 10, worin mindestens ein Katalysator, der aus p-Toluolsulfonsäure, Phosphorpentachlorid (PCl₅) und Phosphorylchlorid (POCl₃) ausgewählt ist, weiter zugegeben wird, wenn die Phenolverbindung (A), der Formaldehyd und die Aminverbindung (B) zusammengemischt werden.

12. Elektrode einer Brennstoffzelle, wobei die Elektrode eine Zusammensetzung nach einem der Ansprüche 1-7 oder ein Polymer nach Anspruch 8 umfasst.

13. Elektrode nach Anspruch 12, worin die Elektrode weiter mindestens eine von einer Gasdiffusionsschicht und einer mikroporösen Schicht und einer Katalysatorschicht umfasst; und
die mindestens eine der Gasdiffusionsschicht, der mikroporösen Schicht und der Katalysatorschicht das Polymer umfasst.

14. Elektrolyt-Membran einer Brennstoffzelle, wobei die Elektrolyt-Membran ein Polymer nach Anspruch 8 umfasst.

15. Brennstoffzelle, die Folgendes umfasst:
eine Kathode;
eine Anode; und
eine Elektrolyt-Membran zwischen der Kathode und der Anode,
worin mindestens eine der Kathode, der Anode und der Elektrolyt-Membran eine Zusammensetzung nach den Ansprüchen 1-7 oder ein Polymer nach Anspruch 8 umfasst.

## Revendications

1. Composition comprenant au moins un d'un composé représenté par la Formule 1 ci-dessous et d'un composé représenté par la Formule 2 ci-dessous : 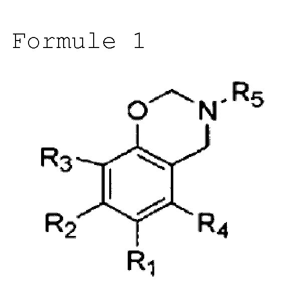 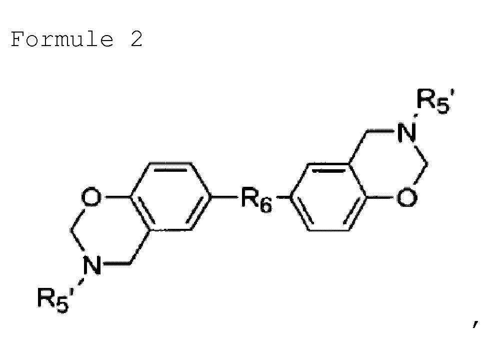
dans lesquelles, dans les Formules 1 et 2, R₁ à R₄ sont chacun indépendamment un atome d'hydrogène, un groupe alkyle en C₁-C₂₀ substitué ou non substitué, un groupe alcoxy en C₁-C₂₀ substitué ou non substitué, un groupe alkényle en C₂-C₂₀ substitué ou non substitué, un groupe alkynyle en C₂-C₂₀ substitué ou non substitué, un groupe aryle en C₆-C₂₀ substitué ou non substitué, un groupe aryloxy en C₆-C₂₀ substitué ou non substitué, un groupe hétéroaryle en C₂-C₂₀ substitué ou non substitué, un groupe hétéroaryloxy en C₂-C₂₀ substitué ou non substitué, un groupe carbocyclique en C₄-C₂₀ substitué ou non substitué, un groupe oxy carbocyclique en C₄-C₂₀ substitué ou non substitué, un groupe hétérocyclique en C₂-C₂₀ substitué ou non substitué, un atome d'halogène, un groupe hydroxyle ou un groupe cyano ;
R₆ est sélectionné parmi le groupe constitué d'un groupe alkylène en C₁-C₂₀ substitué ou non substitué, d'un groupe alkénylène en C₂-C₂₀ substitué ou non substitué, d'un groupe alkynylène en C₂-C₂₀ substitué ou non substitué, d'un groupe arylène en C₆-C₂₀ substitué ou non substitué, d'un groupe hétéroarylène en C₂-C₂₀ substitué ou non substitué, -C(=O)- et -SO₂- ; et
R₅ et R₅' sont chacun indépendamment sélectionnés parmi le groupe constitué d'un groupe représenté par la Formule 3 ci-dessous, d'un groupe représenté par la Formule 4 ci-dessous et de -S=N , 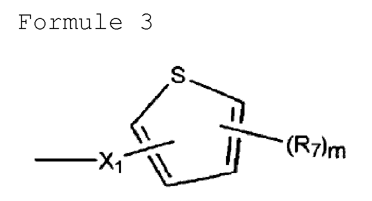 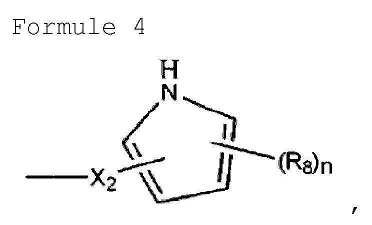
dans lesquelles, dans les Formules 3 et 4, X₁ et X₂ sont chacun indépendamment un groupe alkylène en C₁-C₂₀ substitué ou non substitué, un groupe arylène en C₆-C₂₀ substitué ou non substitué ou un groupe hétéroarylène en C₂-C₂₀ substitué ou non substitué ;
R₇ et R₈ sont chacun indépendamment un atome d'hydrogène, un groupe alkyle en C₁-C₂₀ substitué ou non substitué, un groupe aryle en C₆-C₂₀ substitué ou non substitué, un groupe hétéroaryle en C₂-C₂₀ substitué ou non substitué, un groupe carbocyclique en C₄-C₂₀ substitué ou non substitué, un groupe hétérocyclique en C₂-C₂₀ substitué ou non substitué, un atome d'halogène, un groupe hydroxyle, un groupe amino ou un groupe cyano ; et
m et n sont chacun indépendamment un entier de 1 à 3.

2. Composition selon la revendication 1, dans laquelle R₅ et R₅' sont chacun indépendamment un des groupes représentés par les Formules 5A à 5C ci-dessous : 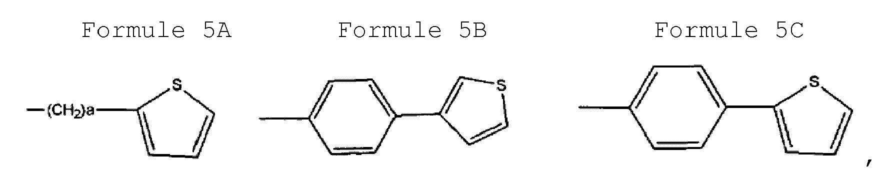 dans lesquelles, dans la Formule 5A, a est un entier de 1 à 5.

3. Composition selon la revendication 1, dans laquelle le composé de la Formule 2 est sélectionné parmi les composés représentés par les Formules 6 à 8 ci-dessous : 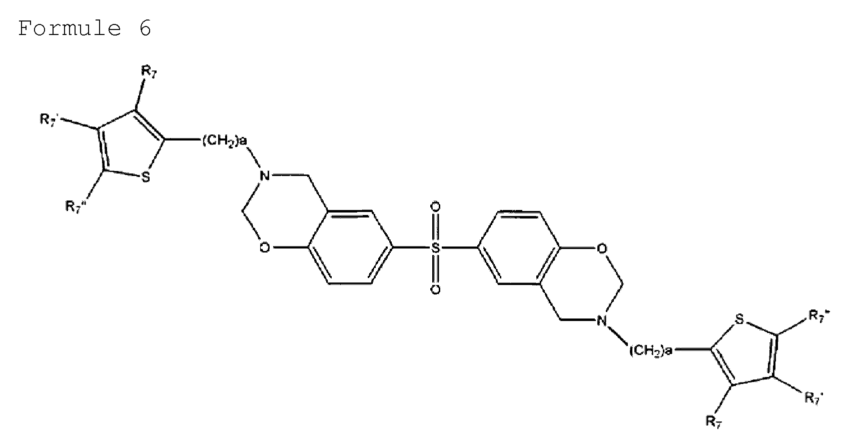 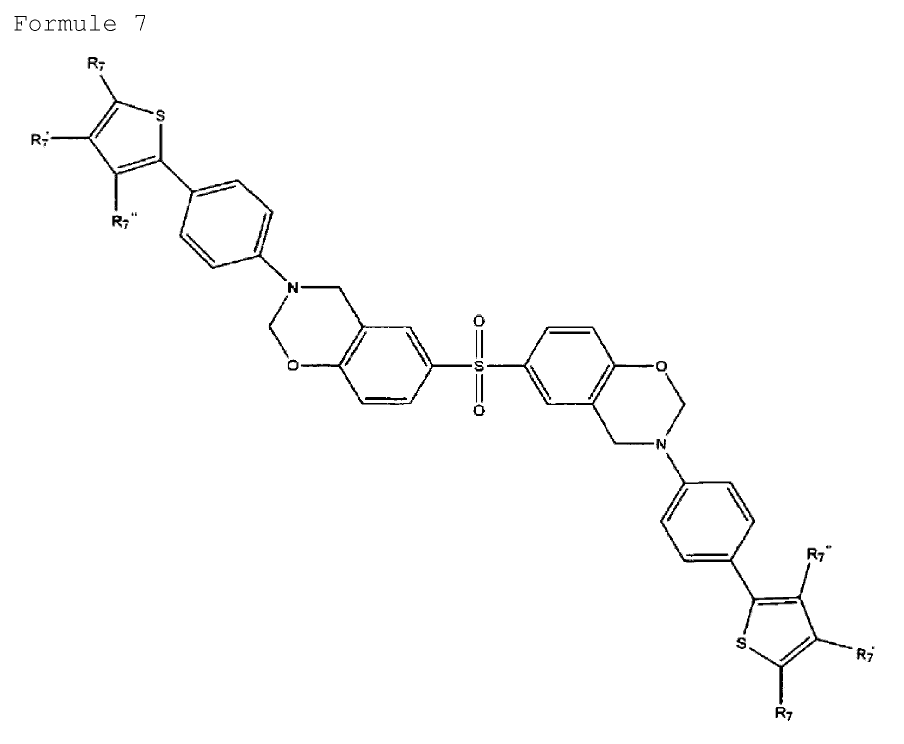 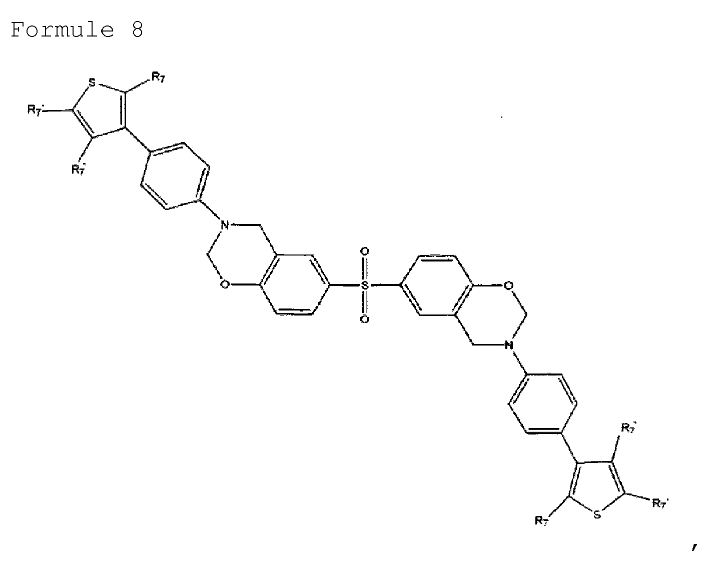
dans lesquelles, dans les Formules 6 à 8, R₇, R₇' et R₇" sont chacun indépendamment un atome d'hydrogène, un groupe alkyle en C₁-C₂₀, un groupe aryle en C₆-C₂₀, un groupe hétéroaryle en C₂-C₂₀, un groupe hétéroaryloxy en C₂-C₂₀, un groupe carbocyclique en C₄-C₂₀, un groupe hétérocyclique en C₂-C₂₀, un atome d'halogène, un groupe hydroxyle ou un groupe cyano ; et
a est un entier de 1 à 5.

4. Composition selon la revendication 1, dans laquelle le composé de la Formule 2 est sélectionné parmi les composés représentés par les Formules 9 à 11 et 19 ci-dessous :

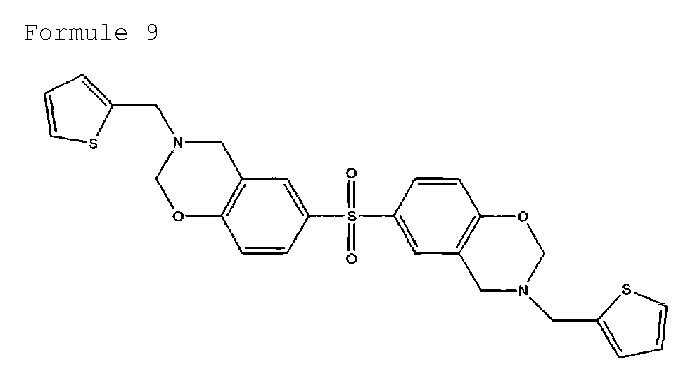

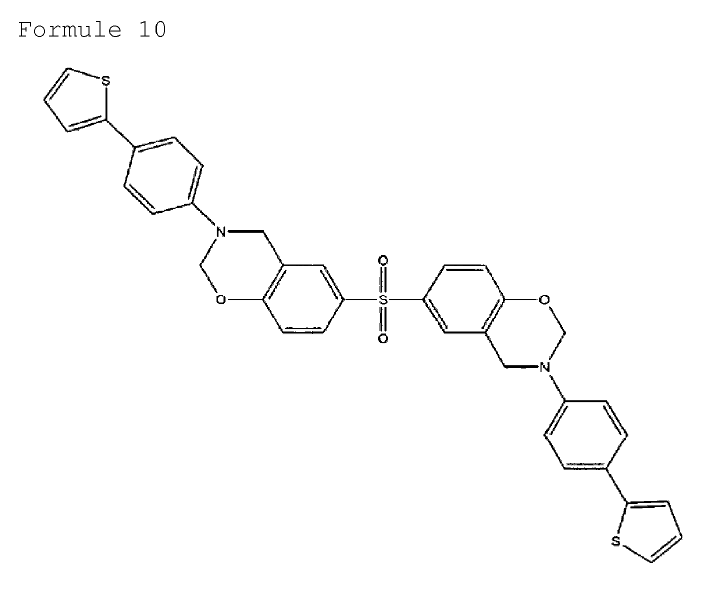

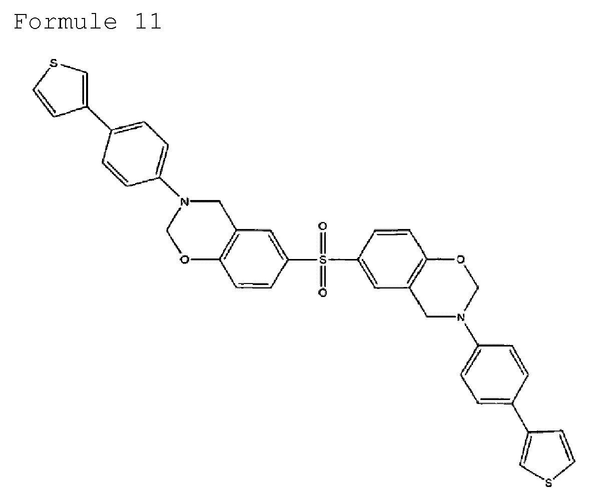

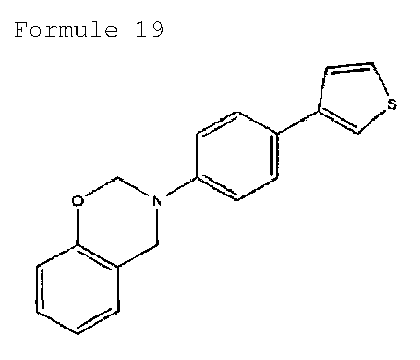

5. Composition selon l'une quelconque des revendications 1 à 4, comprenant en outre un composé pouvant subir une réticulation croisée.

6. Composition selon la revendication 5, dans laquelle le composé pouvant subir une réticulation croisée est au moins un matériau sélectionné parmi le groupe constitué d'un matériau à base de polyazole, d'un polyimide et d'un polyoxazole.

7. Composition selon la revendication 6, dans laquelle le composé pouvant subir une réticulation croisée comprend au moins un composé sélectionné parmi les composés représentés par les Formules 12 à 14 ci-dessous : 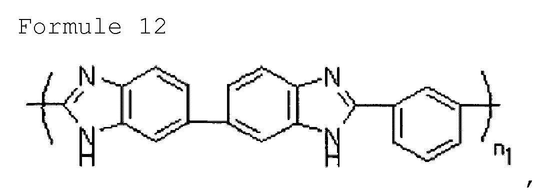
dans laquelle, dans la Formule 12, n₁ est un entier de 10 ou supérieur ; 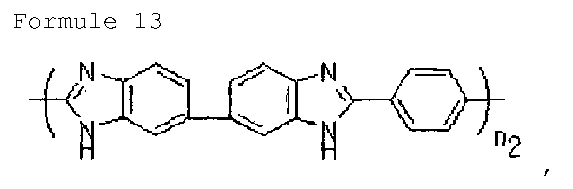
dans laquelle, dans la Formule 13, n₂ est un entier de 10 ou supérieur, 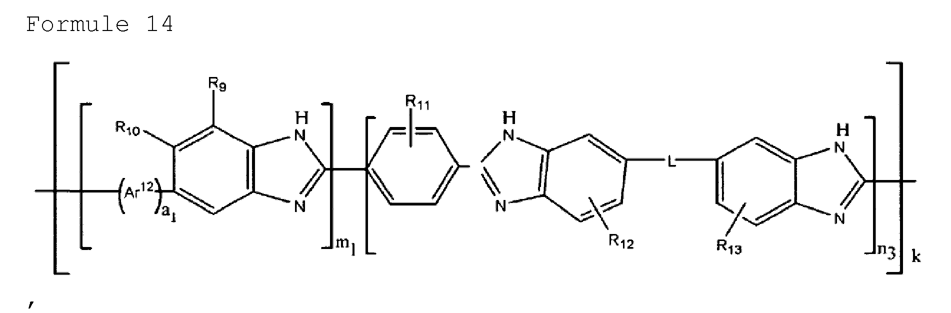
dans laquelle, dans la Formule 14, R₉ et R₁₀ sont chacun indépendamment un atome d'hydrogène, un groupe alkyle en C₁-C₂₀ substitué ou non substitué, un groupe alcoxy en C₁-C₂₀ substitué ou non substitué, un groupe aryle en C₆-C₂₀ substitué ou non substitué, un groupe aryloxy en C₆-C₂₀ substitué ou non substitué, un groupe hétéroaryle en C₃-C₂₀ substitué ou non substitué ou un groupe hétéroaryloxy en C₃-C₂₀ substitué ou non substitué, dans laquelle R₉ et R₁₀ peuvent être liés pour former un groupe carbocyclique en C₄-C₂₀ ou un groupe hétérocyclique en C₃-C₂₀ ;
Ar¹² est un groupe arylène en C₆-C₂₀ substitué ou non substitué ou un groupe hétéroarylène en C₃-C₂₀ substitué ou non substitué ;
R₁₁ à R₁₃ sont chacun indépendamment un substituant mono- ou multi-substitué sélectionné parmi le groupe constitué d'un atome d'hydrogène, d'un groupe alkyle en C₁-C₂₀ substitué ou non substitué, d'un groupe alcoxy en C₁-C₂₀ substitué ou non substitué, d'un groupe aryle en C₆-C₂₀ substitué ou non substitué, d'un groupe aryloxy en C₆-C₂₀ substitué ou non substitué, d'un groupe hétéroaryle en C₃-C₂₀ substitué ou non substitué ou d'un groupe hétéroaryloxy en C₃-C₂₀ substitué ou non substitué ;
L représente un liant ;
m₁ est de 0,01 à 1 ;
a₁ est 0 ou 1 ;
n₃ est un nombre de 0 à 0,99 ; et
k est un nombre de 10 à 250.

8. Polymère qui est un produit de polymérisation de la composition selon l'une quelconque des revendications 1 à 7.

9. Procédé de préparation d'une composition selon l'une quelconque des revendications 1 à 4, le procédé comprenant traiter thermiquement un mélange d'un composé de phénol (A) ci-dessous, de formaldéhyde, d'un composé d'amine (B) ci-dessous, d'un solvant polaire aprotique et d'un solvant organique pour obtenir l'au moins un des composés des Formules 1 et 2 : 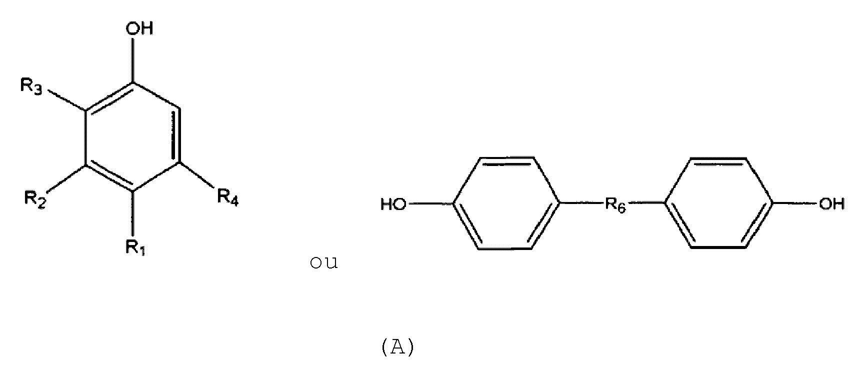
R₅NH₂ ou R₅'NH₂ (B) 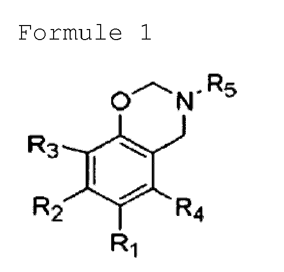 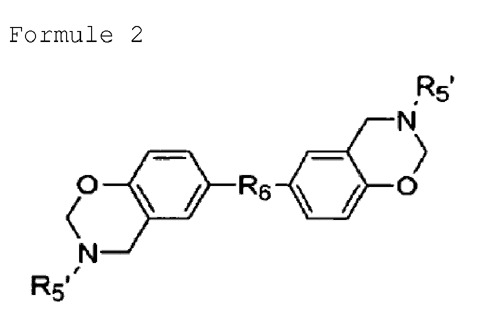
dans lesquelles R₁ à R₄ dans les formules du composé de phénol (A) et R₅, R₅' dans le composé d'amine (B) sont identiques à R₁ à R₄ et R₅, R₅' dans les Formules 1 et 2.

10. Procédé selon la revendication 9, dans lequel le solvant polaire aprotique est au moins un solvant sélectionné parmi le groupe constitué du N,N'-diméthylformamide, N,N'-diméthylacétamide, N-méthylpyrrolidone, tétraméthylènesulfone, 1,2-diméthyl-2-imidazolidinone et N-méthylformamide, et/ou dans lequel le solvant organique comprend au moins un solvant sélectionné parmi le 1,4-dioxane, chloroforme, dichlorométhane, tétrahydrofurane (THF), un solvant à base de benzène de préférence sélectionné parmi le groupe constitué du benzène, toluène et xylène, et un mélange de ceux-ci.

11. Procédé selon la revendication 9 ou 10, dans lequel au moins un catalyseur sélectionné parmi l'acide p-toluènesulfonique, le pentachlorure de phosphore (PCl₅) et le chlorure de phosphoryle (POCl₃) est en outre ajouté lorsque le composé de phénol (A), le formaldéhyde et le composé d'amine (B) sont mélangés ensemble.

12. Electrode d'une pile à combustible, l'électrode comprenant une composition selon l'une quelconque des revendications 1 à 7 ou un polymère selon la revendication 8.

13. Electrode selon la revendication 12, dans laquelle l'électrode comprend en outre au moins une couche parmi une couche de diffusion gazeuse et une couche microporeuse et une couche de catalyseur ; et
l'au moins une couche parmi la couche de diffusion gazeuse, la couche microporeuse et la couche de catalyseur comprend le polymère.

14. Membrane d'électrolyte d'une pile à combustible, la membrane d'électrolyte comprenant un polymère selon la revendication 8.

15. Pile à combustible comprenant :
une cathode ;
une anode ; et
une membrane d'électrolyte entre la cathode et l'anode,
dans laquelle au moins une parmi la cathode, l'anode et la membrane d'électrolyte comprend une composition selon l'une quelconque des revendications 1 à 7 ou un polymère selon la revendication 8.
